# EUROPEAN PATENT SPECIFICATION

(11) **EP 2 617 344 B1**
(45) Date of publication and mention of the grant of the patent: **07.12.2016**
(21) Application number: 12752204.3
(22) Date of filing: 16.02.2012
(51) Int. Cl.: A61B 1/00, A61B 1/005, G06F 3/0338

(54) **BENDING PORTION-EQUIPPED MEDICAL APPARATUS**
MIT EINEM BIEGEABSCHNITT VERSEHENES MEDIZINPRODUKT
DISPOSITIF MÉDICAL DOTÉ D'UNE SECTION DE CAMBRURE

(30) Priority: 28.02.2011 JP 2011042553; 28.02.2011 JP 2011042554
(43) Date of publication of application: 24.07.2013
(73) Proprietor: Olympus Corporation, Tokyo 192-8507 (JP)
(72) Inventor: OKAMOTO, Yasuhiro, Hachioji-shi, Tokyo 192-8507 (JP)
(74) Representative: Schicker, Silvia
(86) International application number: PCT/JP2012/053693
(87) International publication number: WO 2012/117865

(56) References cited:
- JP-A- 6 063 010
- JP-A- 7 302 162
- JP-A- 2003 135 385
- JP-A- 2003 325 437
- JP-A- 2005 279 120
- JP-U- H 062 424
- JP-U- 62 074 803
- US-A1- 2003 092 965
- US-B2- 7 476 952

## Description

### Technical Field

The present invention relates to a bending portion-equipped medical apparatus including a bendable bending portion on a distal end side of an insertion portion and also including an operation element in an operation portion, the operation element being able to be inclined to perform a bending operation of a bending portion.

### Background Art

In recent years, in a medical field or an industrial field, endoscopes including an elongated insertion portion have been used. With the endoscopes in the medical field, the insertion portion is inserted into a body from, e.g., the oral cavity or the anus to perform, e.g., observation. Meanwhile, with the endoscopes in the industrial field, the insertion portion is inserted into, e.g., a piping of a boiler or an inner portion of an engine to perform observation.

In the endoscopes, a bending portion that bends, e.g., upward/downward and rightward/leftward is provided on the distal end side of the insertion portion to enable an observation optical system provided in the distal end portion of the insertion portion to be pointed in a desired direction. Also, in the operation portion provided at a distal end of the insertion portion, a bending operation knob, a bending operation switch or an operation element for performing a bending operation of the bending portion is provided.

The bending operation knob is manually rotated clockwise or counterclockwise to pull or slacken angle wires, whereby the bending portion can be bent. The angle wires are connected to respective predetermined positions in each of the bending operation knob and the bending portion.

Japanese Patent Application Laid-Open Publication No. 05-000125 indicates an endoscope in which a cruciform bending operation switch for performing a bending operation of a bending portion is provided at an operation portion thereof. This endoscope is configured so that, when the bending operation switch is operated, a drive motor is driven and a bending operation wire is pulled or slackened by a drive force of the drive motor, whereby the bending portion is bent.

Meanwhile, examples of the operation element are indicated in, e.g., Japanese Patent Application Laid-Open Publication Nos. 2003-325437 (hereinafter referred to as Document 1), 2008-036355 (hereinafter referred to as Document 2), 06-304124 (hereinafter referred to as Document 3) and 2003-275168 (hereinafter referred to as Document 4).

The endoscope in Document 1 includes a pulling member operating apparatus allowing a bending operation of a bending portion by a tilting operation of an operation instruction lever, which is an operation element, with a very small amount of strength for operation to move a desired pulling member by a desired amount. In this endoscope, a pair of a first tilting state adjusting member and a second tilting state adjusting member, which are movable relative to a plate, are arranged and moved in advance in directions in which the size of a square hole formed in a plate is reduced, whereby a tilting operation range of a bending lever can arbitrarily be adjusted.

Meanwhile, in an operation apparatus in the endoscope in Document 2, a neutral position of an operation element is set with the operation element tilted by a predetermined tilting angle toward a grasping portion for enabling easy operation even when the operation element is moved in a direction away from the grasping portion. The tilting angle is determined so that an angle formed by a center line of the operation element and a center line of the grasping portion is smaller than 90° at the neutral position. Consequently, even at a maximum tilting position of the operation element where the operation element is tilted to the maximum in the direction away from the grasping portion, an operator easily makes a finger of his/her hand grasping the grasping portion reach the operation element, enabling easy operation.

Also, Document 3 indicates an endoscope in which a joystick switch for performing a bending operation of a bending portion is provided at an operation portion. This endoscope is configured so that an angle lever, which is an operation element of the joystick switch, is inclined in multiple directions by a thumb to perform a bending operation of the bending portion in a desired direction, whereby bending operation wires are pulled and slackened to bend the bending portion.

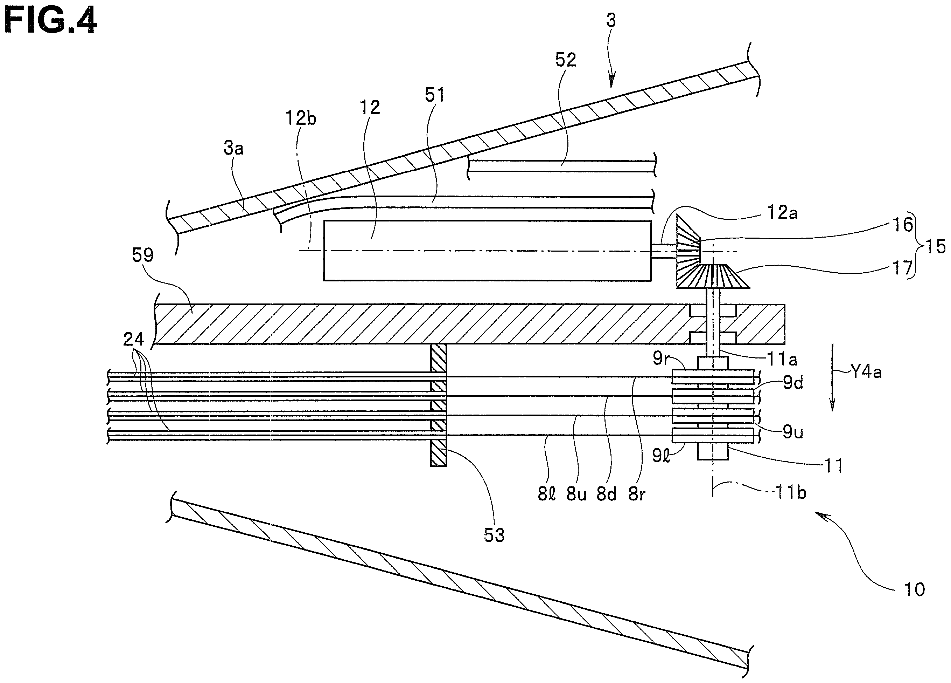

Also, in a motor-driven bending endoscope apparatus in Document 4, Fig. 4(b) indicates an endoscope in which an operation switch includes a stick section, which is an operation element, and a tilting direction of the stick section is a bending direction of the bending portion, and during a tilting operation, a bending motor is rotated at a fixed speed to change the bending state. In this endoscope, the stick section is brought into arc motion, enabling pivoting motion of the bending portion.

However, in Document 1, the first and second tilting state adjusting members are moved in the directions in which the size of a square hole is reduced, whereby the tilting operation range of a bending lever is adjusted. Therefore, there is a possibility that the maximum bending angle of the bending portion becomes small and functions of the bending portion cannot be used maximally. In an attempt to configure an endoscope capable of maximally using the functions of the bending portion by incorporating the technique in Document 2 into the technique in Document 1, the shape of the operation portion has to be changed in order to set a neutral position of the bending lever so as to be tilted by a predetermined tilting angle.

In addition, in Documents 3 and 4, the operator places the cushion of the thumb, for example, on the end portion of the operation element, to tilt the operation element, and performs an operation for bending the bending portion. Therefore, when the operator brings the operation element into arc motion, there is a possibility that the thumb mistakenly falls off from the end portion of the operation element. In addition, when the operator tilts the operation element in a standing state to bend the bending portion, as the tilting angle becomes larger, the operator is likely to get his/her thumb away from the end portion of the operation element. Therefore, during an examination, if the operator continues an operation while being careful not to get his/her thumb away from the end portion of the operation element, the tilting angle of the operation element becomes small, and as a result, the bending angle of the bending portion becomes small.

The present invention has been achieved in view of the above-described circumstances, and it is an object of the present invention to provide a bending portion-equipped medical apparatus including an operation element with excellent operability which stands with respect to a grasping surface of the operation portion, and which is capable of stably performing an operation for arc motion and increasing a tilting operation range while preventing the fingers from falling off from the operation element.

US 2003/092965 discloses an electric bending endoscope where a desired traction member is moved by a desired amount to bend a bending portion by slantingly operating an operation instructing lever with a small amount of operation force.

US 7476952 discloses a force input control device suitable for high-volume applications such as cell phones, portable gaming devices and other handheld electronic devices is disclosed. The device comprises a button, which has multiple ergonomical designs, is mechanically coupled to the force-transferring element of the package for providing an interface with the external force.

### Disclosure of Invention

The invention is defined in independent claim 1 and the dependent claims.

### Brief Description of the Drawings

Figs. 1-12 relate to a first embodiment of the present invention, and Fig. 1 is a diagram illustrating an endoscope, which is an example of a bending portion-equipped medical apparatus in which an operation element included in a pulling member operating apparatus is provided in a standing manner at an operation portion;
Fig. 2 is a diagram illustrating a configuration of a pulling member operating apparatus with a motor and a pulley incorporated in an operation portion including a grasping portion and an operation portion body;
Fig. 3 is a diagram illustrating a rotating body;
Fig. 4 is a diagram mainly illustrating configurations of the motor and the pulley in the pulling member operation apparatus as viewed from an arrow Y4 in Fig. 2;
Fig. 5 is a diagram mainly illustrating attachment path setting members and a hanging frame in the pulling member operation apparatus as viewed from an arrow Y4 in Fig. 2;
Fig. 6 is a diagram illustrating a finger contact portion configured in the form of a pentahedron;
Fig. 7 includes a top view of the finger contact portion illustrated in Fig. 6 and a front view of a fourth operation surface;
Fig. 8 is a cross-sectional diagram along arrows Y8-Y8 line in Fig. 7;
Fig. 9 is a cross-sectional diagram along arrows Y9-Y9 line in Fig. 7;
Fig. 10 is a diagram illustrating a cross-sectional configuration of a finger contact portion including a core member and a covering body;
Fig. 11 is a diagram illustrating a cross-sectional configuration of a finger contact portion including a core member and a covering body having a double-layer structure;
Fig. 12 is a diagram illustrating a cross-sectional configuration of a finger contact portion including a core member and an elastic sheet provided on predetermined surfaces of the core member;
Fig. 13 is a diagram illustrating a finger contact portion according to a second embodiment of the present invention, the finger contact portion being configured in the form of a hexahedron;
Figs. 14-22 relate to a modification of the finger contact portion configured in the form of a hexahedron, and Fig. 14 is a top view of a finger contact portion including a recess portion at a top face as a finer support portion and also including a recess portion at each operation surface;
Fig. 15 is a side view of the finger contact portion as viewed from an arrow Y 15 in Fig. 14;
Fig. 16 is a back view of the finger contact portion as viewed from an arrow Y 16 in Fig. 14;
Fig. 17 is a cross-sectional diagram along arrows Y17-Y17 line in Fig. 14;
Fig. 18 is an enlarged view of the part indicated by an arrow Y18 in Fig. 17;
Fig. 19 is a cross-sectional diagram along arrows Y19-Y19 line in Fig. 14;
Fig. 20 is a cross-sectional diagram along arrows Y20-Y20 line in Fig. 14;
Fig. 21 is a cross-sectional diagram along arrows Y21-Y21 line in Fig. 14;
Fig. 22 is a diagram illustrating a state in which a side part of a thumb is put on a first operation surface and a state in which the other side part of the thumb is put on a second operation surface;
Figs. 23-25 relate to diagrams illustrating another modification of the finger contact portion, and Fig. 23 is a top diagram illustrating a configuration of a finger contact portion;
Fig. 24 is a cross-sectional diagram along arrows Y24-Y24 line in Fig. 23;
Fig. 25 is a cross-sectional diagram along arrows Y25-Y25 line in Fig. 23;
Figs. 26-27B relate to diagrams illustrating still another modification of the finger contact portion, and Fig. 26 is a diagram illustrating a configuration of a finger contact portion in which a finger rest body and a recess portion-provided finger support member are separated from each other;
Fig. 27A is a diagram illustrating an operation of the finger contact portion, i.e., a diagram illustrating a state in which a finger is put on a finger support recess portion to operate the recess portion-provided finger support member in an arrow F direction; and
Fig. 27B is a diagram illustrating a state in which the recess portion-provided finger support member slides relative to the finger rest body.

### Best Mode for Carrying Out the Invention

Hereinafter, embodiments of the present invention will be described with reference to the drawings.

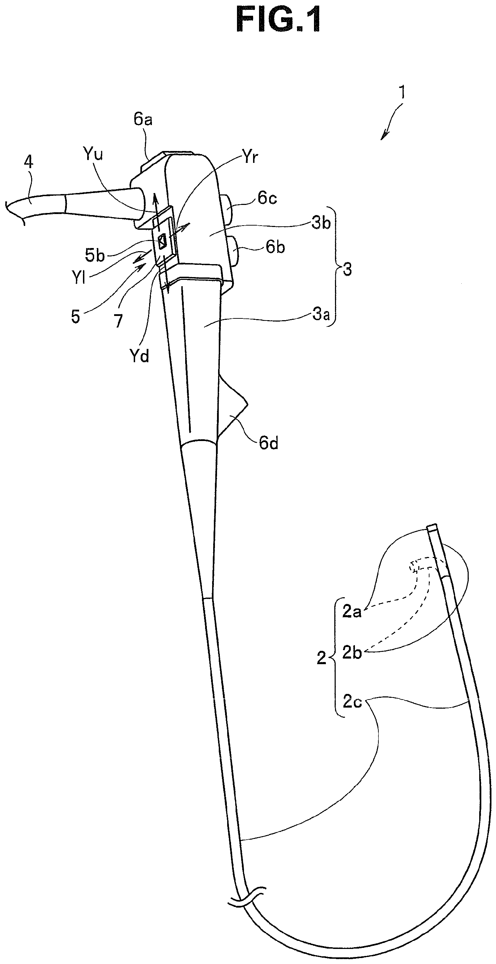

A first embodiment of the present invention will be described with reference to Figs. 1-12.

In the present embodiment, a bending portion-equipped medical apparatus is an endoscope. As illustrated in Figs. 1-6, an endoscope 1 according to the present embodiment includes an elongated insertion portion 2, an operation portion 3 consecutively connected to a proximal end of the insertion portion 2, and a universal cord 4 extending from a side part of the operation portion 3.

The insertion portion 2 includes a distal end portion 2a, a bending portion 2b and a flexible tube portion 2c, which are consecutively connected in this order from the distal end side. In the distal end portion 2a, an image pickup apparatus (not illustrated) including an image pickup device is incorporated. The bending portion 2b is configured so as to be bendable, for example, upward/downward and rightward/leftward. The flexible tube portion 2c has flexibility and has a long length.

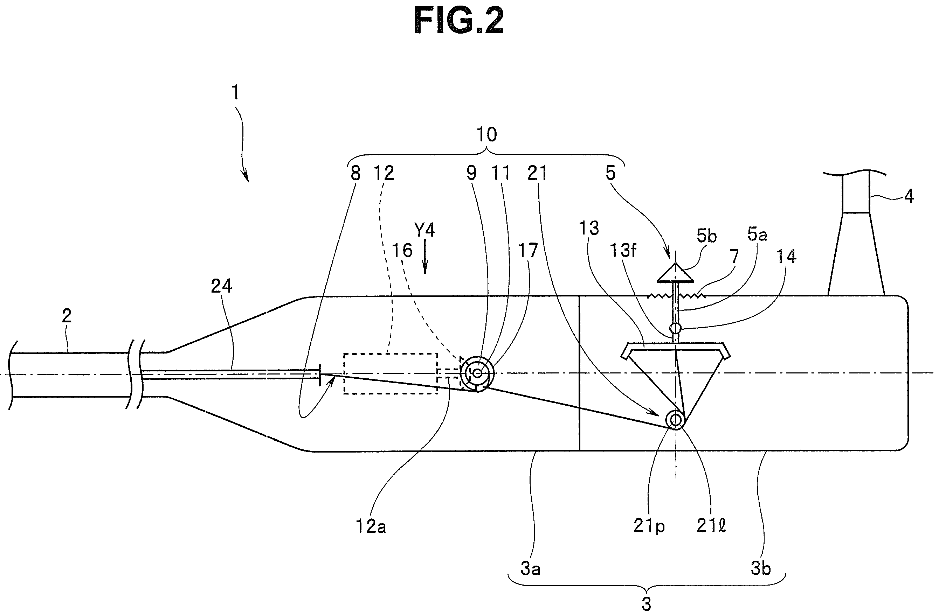

As illustrated in Figs. 1 and 2, the operation portion 3 includes a grasping portion 3a and an operation portion body 3b. The grasping portion 3a is consecutively connected to the insertion portion 2, and the operation portion body 3b is consecutively connected to the grasping portion 3a. A longitudinal axis of the grasping portion 3 a and an insertion axis of the insertion portion 2 are common or parallel to each other. At a position corresponding to a part on the distal end side of the operation portion body 3b where a largest vacant space is provided, an operation element 5 for performing an operation to bend the bending portion 2b is provided. A longitudinal axis of the operation portion body 3b (also referred to as a longitudinal axis of the operation portion 3) and the longitudinal axis of the grasping portion 3a are common to or parallel to each other.

The operation element 5 is provided perpendicular to the longitudinal axis of the operation portion 3 from an operation element projecting opening (not illustrated), which is an opening provided at a surface of the operation portion body 3b.

As indicated by arrows Yu, Yd, Yl and Yr in Fig. 1, the bending portion 2b is configured so as to bend according to a tilting operation including a tilting direction and a tilting angle of the operation element 5. More specifically, the bending portion 2b bends in directions such as upward, rightward, downward, leftward and a direction between upward and rightward upon the later-described bending operation wires (hereinafter abbreviated as bending wires) being pulled/slackened by means of a tilting operation of the operation element 5.

In the present embodiment, the bending portion 2b is configured so as to bend in four directions, i.e., upward, downward, leftward and rightward. However, the bending portion 2b may be configured so as to bend upward and downward. The aforementioned symbols u, d, l and r indicate upward, downward, leftward and rightward, which are bending directions of the bending portion 2b. In the below description, for example, reference numeral 8u denotes a bending wire for upward bending, and reference numeral 9d denotes a rotating body for downward bending. In the figures, the small letter "1" is indicated in cursive script to make a distinction between the small letter "l" and the numeral "1."

Also, as illustrated in Fig. 1, in the sheath of the operation portion body 3b, a switch 6a, air/water feeding button 6b and a suction button 6c are provided at predetermined positions in addition to the operation element 5. The switch 6a is provided to, for example, give instructions to perform various image pickup operations of the image pickup apparatus provided in the distal end portion 2a. Also, in the sheath of the grasping portion 3a, a channel insertion opening 6d that is in communication with a treatment instrument channel (not illustrated) is provided.

In the present embodiment, when an operator grasps the grasping portion 3 a of the operation portion 3 by his/her left hand as with a conventional endoscope, the operation element 5 is provided at a position where the operation element 5 is operated by the thumb of the grasping hand of the operator, the air/water feeding button 6b and the suction button 6c are provided at positions where the air/water feeding button 6b and the suction button 6c are operated by fingers other than the thumb of the grasping hand of the operator, and the switch 6a is provided at a position where the switch 6a can be operated by the thumb or another finger of the grasping hand of the operator.

Reference numeral 7 in Figs. 1 and 2 indicates a cover member. The cover member 7 occludes the operation element projecting opening in a water-tight manner and is in close contact with a shaft portion 5a, and holds the operation element 5 in a tiltable manner.

In the universal cord 4, e.g., a signal cable, an electric wire, a light guide fiber bundle, an air feeding tube, a water feeding tube and a suction tube are inserted. The signal cable is connected to the image pickup apparatus. The electric wire supplies power to the later-described motor (see reference numeral 12 in Fig. 2). The light guide fiber bundle conveys illuminating light from a light source apparatus.

As illustrated in Fig. 2, in the operation portion 3, a pulling member operation apparatus 10 is provided. The pulling member operation apparatus 10 mainly includes four bending wires 8, an elongated pulley 11 on which four rotating bodies 9 are disposed, a motor 12 that is drive means, a substantially-cruciform hanging frame 13, the operation element 5, and a guide roller set 21 including a plurality of guide rollers described later.

The bending wires 8 are pulling members. Parts of the respective wires 8 are wound on the respective rotating bodies 9. The motor 12 has a drive force that rotates a predetermined rotating body 9 provided on the pulley 11 with predetermined torque during a bending operation. The hanging frame 13 includes wire attachment portions to which respective proximal end portions of the respective wires 8 are connected. The shaft portion 5a of the operation element 5 is connected to the hanging frame 13 in an integrated manner. The plurality of guide rollers in the guide roller set 21 provide wire travel path changing members that change travel paths of the four wires 8 inside the operation portion 3.

In Fig. 4, reference numeral 51 denotes a signal cable, reference numeral 52 denotes a light guide cable, reference numeral 53 denotes a coil pipe stopper, and reference numeral 59 denotes a partition plate. In the present embodiment, the gravity center of the operation portion 3 is positioned inside the grasping portion 3a.

The four bending wires 8 include a pair of a bending wire for upward bending (hereinafter, referred to as an upward bending wire) 8u and a bending wire for downward bending (hereinafter, referred to as a downward bending wire) 8d for upward/downward bending operation, and a pair of a bending wire for leftward bending (hereinafter, referred to as a leftward bending wire) 81 and a bending wire for rightward bending (hereinafter, referred to as a rightward bending wire) 8r for leftward/rightward bending operation.

In the present embodiment, a longitudinal axis of the pulley 11 and a longitudinal axis of the motor 12 intersect each other.

In particular, a drive shaft of the motor 12 is arranged at a position determined in advance inside the grasping portion 3a so that the drive shaft of the motor 12 is parallel to the longitudinal axis of the grasping portion 3a. A motor shaft 12b of the motor 12 and a pulley shaft 11b that is a rotational axis of the pulley 11, are set to be perpendicular to each other. Also, the pulley 11 and the motor 12 are arranged with the partition plate 59 therebetween in different spaces inside the operation portion 3, which are separated by the partition plate 59.

The drive force of the motor 12 is transmitted to the pulley 11 by a drive force transmission mechanism portion 15. The drive force transmission mechanism portion 15 includes a first bevel gear 16 and a second bevel gear 17.

The first bevel gear 16 is fixed to the shaft portion 12a of the motor 12 in an integrated manner, and the second bevel gear 17 is fixed to the shaft portion 11a of the pulley 11 in an integrated manner. With this configuration, a drive force of the motor 12 is transmitted to the shaft portion 11a via the bevel gears 16 and 17, whereby the pulley 11 rotates with reference to the shaft.

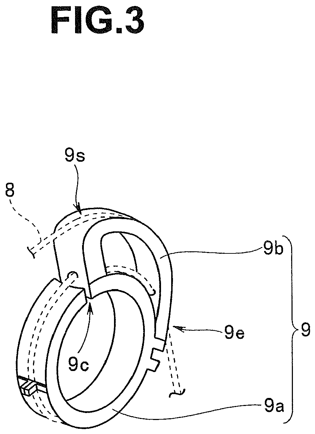

The rotating bodies 9 are elastically deformable, and for example, as illustrated in Fig. 3, each include a ring-like portion 9a and a rotation amount adjusting portion 9b. In the ring-like portion 9a of each rotating body 9, a gap 9c is formed. In each of the ring-like portion 9a and the rotation amount adjusting portion 9b, a non-illustrated wire guiding portion is formed. The wire guiding portion is configured to have a shape determined in advance so as to smoothly guide the relevant wire 8 from a winding start position 9s to a winding end position 9e. Four rotating bodies 9u, 9d, 91 and 9r are arranged in a predetermined loose fit state on an outer circumferential face of the pulley 11, and independently rotate from one another.

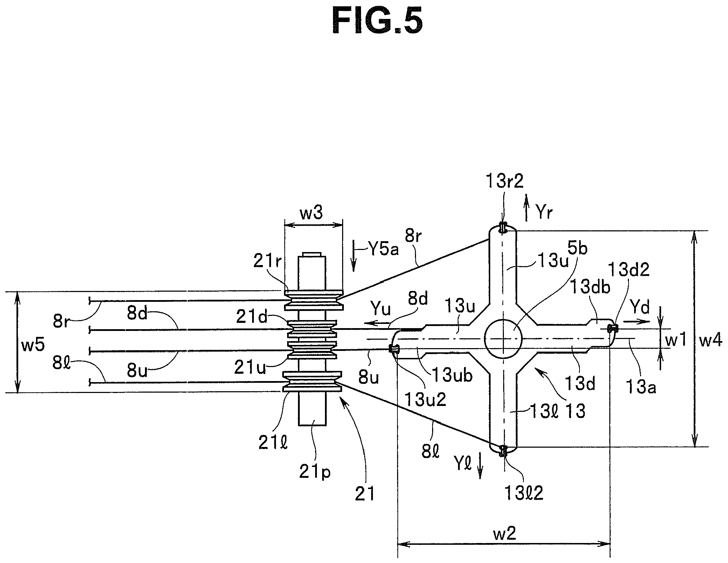

The hanging frame 13 is arranged in a predetermined position inside the vacant space on the distal end side of the operation portion body 3b. As illustrated in Fig. 5, the hanging frame 13 includes four frames 13u, 13d, 131 and 13r and is configured to be substantially cruciform. A frame for upward bending (hereinafter, referred to as an upward frame) 13u and a frame for downward bending (hereinafter, referred to as a downward frame) 13d, which correspond to the pair of bending wires 8u and 8d, are arranged on a straight line with the shaft portion 5a therebetween. At an end portion of the upward frame 13u, an upward wire attachment portion 13u2 is provided, and at an end portion of the downward frame 13d, a downward wire attachment portion 13d2 is provided.

Meanwhile, a frame for leftward bending (hereinafter, referred to as a leftward frame) 131 and a frame for rightward bending (hereinafter, referred to as a rightward frame) 13r, which correspond to the pair of bending wires 81 and 8r, are arranged perpendicular to a center line 13a of the upward and downward frames (hereinafter, referred to as a frame center line) and on a straight line with the shaft portion 5a therebetween. At an end portion of the leftward frame 131, a leftward wire attachment portion 1312 is provided, and at an end portion of the rightward frame 13r, a rightward wire attachment portion 13r2 is provided.

The upward frame 13u includes an upward frame distal end curved portion 13ub at the end portion thereof, the upward-frame distal end curved portion 13ub being flexed in one direction relative to the frame center line 13a, and the down frame 13d includes a down frame distal end curved portion 13db at the end portion thereof, the down frame distal end curved portion 13db being flexed in another direction relative to the frame center line 13a.

Then, the upper wire attachment portion 13u2 is provided at the upward frame distal end curved portion 13ub, and the downward wire attachment portion 13d2 is provided at the down frame distal end curved portion 13db. A distance w1 in a direction perpendicular to the longitudinal direction of the operation portion 3 between the upward wire attachment portion 13u2 and the downward wire attachment portion 13d2 is set to a predetermined dimension.

The upward frame 13u and the upward wire attachment portion 13u2, etc., are set with taking the tilting direction of the operation element 5 and a bending direction of the bending portion 2b into account. In the present embodiment, when the operation element 5 is tilted in the arrow Yu direction in Fig. 1, the upward wire attachment portion 13u2 is swung and inclined in the arrow Yu direction in Fig. 5, whereby the bending portion 2b bends upward. Also, similarly, when the operation element 5 is tilted in the arrow Yd direction in Fig. 1, the downward wire attachment portion 13d2 is swung and inclined in the arrow Yd direction in Fig. 5, whereby the bending portion 2b bends downward, and when the operation element 5 is tilted in the arrow Yl direction in Fig. 1, the leftward wire attachment portion 1312 is swung and inclined in the arrow Yl direction in Fig. 5, whereby the bending portion 2b bends leftward, and when the operation element 5 is tilted in the arrow Yr direction in Fig. 1, the rightward wire attachment portion 13r2 is swung and inclined in the arrow Yr direction in Fig. 5, whereby the bending portion 2b bends rightward.

In the present embodiment, the hanging frame 13 is arranged at the predetermined position inside the operation portion 3 so that the frame center line 13a and the longitudinal direction of the grasping portion 3a are parallel to each other.

As illustrated in Figs. 2 and 5, the guide roller set 21 includes a roller shaft 21p, and four guide rollers 21u, 21d, 211 and 21r. The roller shaft 21p is a support, and has, for example, a circular columnar shape. The four guide rollers 21u, 21d, 211 and 21r are rotatably arranged on the roller shaft 21p.

The four guide rollers 21u, 21d, 211 and 21r correspond to the four bending wires 8u, 8d, 81 and 8r, respectively. The four guide rollers 21u, 21d, 211 and 21r are provided at a predetermined distance from the pulley 11 and the hanging frame 13. The four guide rollers 21 u, 21d, 211 and 21r are attachment path setting members that guide the four bending wires 8u, 8d, 81 and 8r to the wire attachment portions 13u2, 13d2, 1312 and 13r2 of the hanging frame 13, respectively.

The roller shaft 21p is arranged at a predetermined position immediately below the shaft portion 5a and perpendicular to the longitudinal axis of the grasping portion 3 a. A center of the roller shaft 21p is positioned on a center axis of the shaft portion 5a in a standing state.

The respective bending wires 8u, 8d, 81 and 8r are subjected to travel path change by the guide rollers 21u, 21d, 211 and 21r, and then reach the upward wire attachment portion 13u2, the downward wire attachment portion 13d2, the leftward wire attachment portion 1312 and the rightward wire attachment portion 13r2 of the hanging frame 13, respectively.

The guide roller 21 will be described with reference to Fig. 5.

In Fig. 5, for illustration of a positional relationship between the respective bending wires 8u, 8d, 81 and 8r and the respective wire attachment portions 13u2, 13d2, 1312 and 13r2, the position of the hanging frame 13 is shifted to the right in the figure relative to the roller shaft 21p.

As illustrated in Fig. 5, the four guide rollers 21 u, 21 d, 211 and 21 r are arranged in the order of the guide rollers 21 r, 21 d, 21 u and 211 relative to the roller shaft 21p as indicated by arrow Y5a in Fig. 5.

The guide rollers 21r and 211 arranged at opposite ends of the roller shaft 21p, and guide rollers 21u and 21 d arranged on the inner side of the guide rollers 21r and 211 with a center of the roller shaft 21p therebetween have different diameter dimensions or width dimensions. At least the width dimension of the guide rollers 211 and 21r are set to be larger than the width dimension of the guide rollers 21u and 21d.

Where a maximum outer diameter of the guide rollers 211, 21r, 21 u and 21d is w3 and a distance in the longitudinal axis direction of the operation portion 3 between the upward wire attachment portion 13u2 and the downward wire attachment portion 13d2 is w2, a relationship of w2 > w3 is set between the distance w2 and the maximum outer diameter w3.

Furthermore, a distance between a center of the guide roller 21u and a center of the guide roller 21d is set to a distance w1 between the upward wire attachment portion 13u2 and the downward wire attachment portion 13d2.

Furthermore, a relationship of w4 > w5 is set between the distance w4 between the leftward wire attachment portion 1312 and the rightward wire attachment portion 13r2 and the distance w5 between an outer end of the leftward guide roller 211 and an outer end of the right guide roller 21r, which are arranged on the roller shaft 21p.

The four rotating bodies 9 arranged on the pulley 11 are arranged in the order of the rotating bodies 9r, 9d, 9u and 91 as indicated by the arrow Y4a in Fig. 4.

Here, the travel paths of the respective bending wires 8u, 8d, 81 and 8r inside the operation portion 3 will be described with reference to Figs. 2, 4 and 5.

As illustrated in Fig. 5, respective proximal end portions of the four bending wires 8u, 8d, 81 and 8r are fixed to the respective wire attachment portions 13u2, 13d2, 1312 and 13r2, which are predetermined positions, of the hanging frame 13.

Meanwhile, respective distal end portions of the respective bending wires 8u, 8d, 81 and 8r are fixed at positions corresponding to upward, downward, leftward and rightward bending of non-illustrated distal end bending pieces included in the bending portion 2b. The distal end bending pieces are distal end pieces included in a farthest distal end of a bending portion set configured so that a plurality of non-illustrated bending pieces included in the bending portions 2b are joined so as to bend upward, downward, leftward and rightward.

The respective bending wires 8u, 8d, 81 and 8r are inserted into guides 24 corresponding to the respective wires 8u, 8d, 81 and 8r inside the insertion portion 2 in such a manner that the respective bending wires 8u, 8d, 81 and 8r can freely advance and retract, the guides 24 each being formed using a coil pipe, the coil pipe being made of, for example, a metal and including a through hole.

As illustrated in Figs. 2, 4 and 5, the respective bending wires 8u, 8d, 81 and 8r fixed to the distal end bending pieces extend to the inside of the operation portion 3 via the guides 24.

The respective bending wires 8u, 8d, 81 and 8r are wound on the respective rotating bodies 9u, 9d, 91 and 9r arranged on the pulley 11. In other words, the respective bending wires 8u, 8d, 81 and 8r are wound on the rotating bodies 9u, 9d, 91 and 9r from the respective winding start positions 9s of the corresponding rotating bodies 9u, 9d, 91 and 9r so that the respective bending wires 8u, 8d, 81 and 8r each enter a predetermined slackened state. Subsequently, the respective bending wires 8u, 8d, 81 and 8r are guided out toward the respective guide rollers 21u, 21d, 211 and 21r from the respective winding end position 9e of the respective rotating bodies 9u, 9d, 91 and 9r.

The respective bending wires 8u, 8d, 81 and 8r guided out from the respective rotating bodies 9u, 9d, 91 and 9r are guided to the respective guide rollers 21u, 21d, 211 and 21r, and subjected to wire travel path change and further guided to the wire attachment portions 13u2, 13d2, 1312 and 13r2 included in the hanging frame 13. Then, the respective proximal end portions of the respective bending wires 8u, 8d, 81 and 8r are fixed to the wire attachment portions 13u2, 13d2, 1312 and 13r2.

As described above, the width dimension of the guide rollers 211 and 21r is set to be larger than the width dimension of the guide rollers 21u and 21d, and the distance w4 is set to be larger than the distance w5. Consequently, the bending wires 81 and 8r smoothly pass on the guide rollers 211 and 21r and are guided to the wire attachment portions 1312 and 13r2.

The shaft portion 5a of the operation element 5 and a frame projection portion 13f that is a center axis of the hanging frame 13, are concentrically attached and fixed to each other via a universal joint 14 pivotally disposed in a non-illustrated frame. When the shaft portion 5a of the operation element 5 is in a standing state as illustrated in the figures, the respective bending wires 8u, 8d, 81 and 8r extending from the guide rollers 21u, 21 d, 211 and 21r toward the hanging frame 13 are all in a predetermined slackened state.

Reference numeral 5b denotes a finger contact portion. The finger contact portion 5b is fixed to a distal end of the shaft portion 5a in an integrated manner.

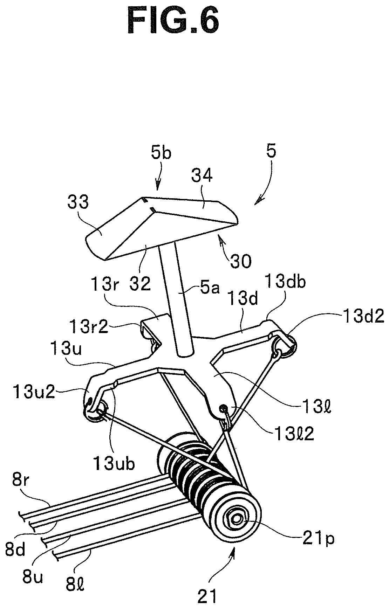

A configuration of the finger contact portion 5b will be described with reference to Figs. 6-9.

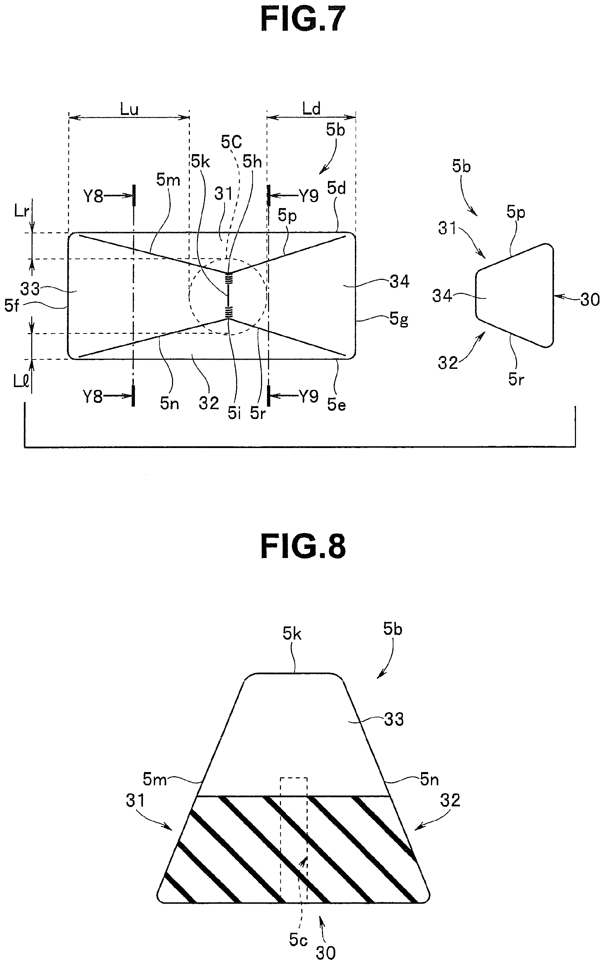

The finger contact portion 5b illustrated in the figures includes an elastic member having a predetermined resilient property at a part thereof or include an elastic member in its entirety. As illustrated in Figs. 6 and 7, the finger contact portion 5b is a pentahedron including a bottom face 30, a first operation surface 31, a second operation surface 32, a third operation surface 33 and a fourth operation surface 34.

The bottom face 30 has a quadrangular shape formed by four sides set to have predetermined lengths, and for example, a rectangular shape as illustrated in Fig. 7.

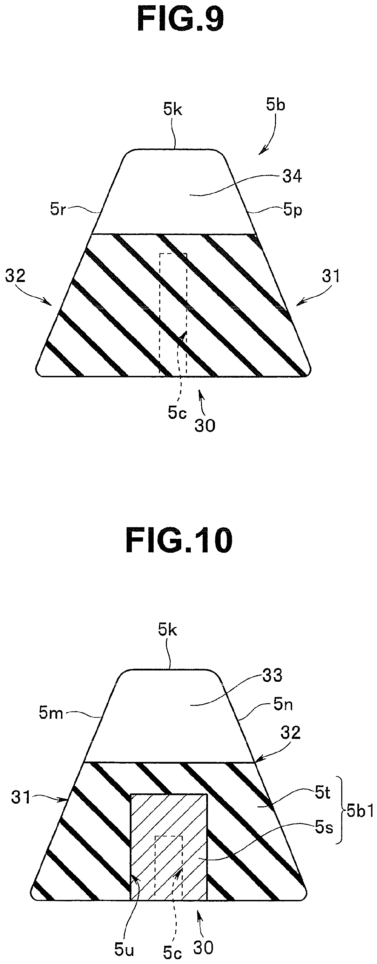

The bottom face 30 is an attachment surface including an attachment portion 5c indicated by the dashed lines in Figs. 8 and 9. The attachment portion 5c is, for example, a hole or a screw hole including a female thread. An end portion of the shaft portion 5a is arranged in the attachment portion 5c. The finger contact portion 5b and the shaft portion 5a are attached to each other in an integrated manner by means of, for example, bonding or screw connection. In the present embodiment, the finger contact portion 5b and the shaft portion 5a are fixed by bonding.

As illustrated in Figs. 6 and 7, the first operation surface 31 is an inclined surface having a triangular shape with a first side 5d of the bottom face 30 as a bottom face of the triangular shape. The second operation surface 32 is an inclined surface having a triangular shape with a second side 5e facing the first side 5d as a bottom face of the triangular shape. The third operation surface 33 is an inclined surface having a quadrangular shape with a third side 5f, which is one of the third side 5f and a fourth side 5g intersecting the first side 5d of the bottom face 30, as one side of the quadrangular shape. The fourth operation surface 34 is an inclined surface having a quadrangular shape with the fourth side 5g facing the third side 5f as one side of the quadrangular shape. The third operation surface 33 and the fourth operation surface 34 are arranged along an insertion axis direction. The third operation surface 33 is positioned on the distal end side of the endoscope 1, and the fourth operation surface is positioned on the proximal end side of the endoscope 1.

In the present embodiment, a ridge line 5k connecting a first apex 5h facing the first side 5d of the first operation surface 31 and a second apex 51 facing the second side 5e of the second operation surface 32 and other ridge lines 5m, 5n, 5p and 5r are each chamfered to have a curved surface. Also, the respective sides 5d, 5e, 5f and 5g are each chamfered to have a curved surface.

Also, in the present embodiment, the third operation surface 33 and a surface of the fourth operation surface 34 include antislip-finished surfaces. Examples of the antislip-finished surfaces include a pearskin-finished surface or an undulating surface in which rows of, e.g., projections and recesses are regularly arranged.

The shape of the bottom face 30 is not limited to a rectangular shape, and may be, e.g., a trapezoidal shape in which the third side 5f is longer than the fourth side 5g or conversely, a trapezoidal shape in which the fourth side 5g is longer than the third side 5f.

Also, in the present embodiment, the first operation surface 31 is an operation surface for rightward bending, on which a side portion of a thumb is put. The second operation surface 32 is an operation surface for leftward bending, on which the other side portion of the thumb is put. The third operation surface 33 is an operation surface for upward bending, on which the cushion of the thumb is put. The fourth operation surface 34 is an operation surface for downward bending, on which the cushion of the thumb is put as with the third operation surface 33.

Here, an operation when an operator performs an operation to bend the bending portion 2b, for example, upward will be described.

With the grasping portion 3a grasped by his/her left hand, the operator puts the cushion of the thumb on the third operation surface 33 included in the finger contact portion 5b of the operation element 5. The operator then performs an operation to tilt the shaft portion 5a in the arrow Yu direction in Fig. 1. Then, with the tilting operation of the operation element 5, the hanging frame 13 is inclined, the upward bending wire 8u fixed to the upward wire attachment portion 13u2 gradually changes from a slackened state to a pulled state. Meanwhile, the other bending wires 8d, 81 and 8r change to a further slackened state.

Accordingly, from among the respective bending wires 8u, 8d, 81 and 8r wound on the rotating bodies 9u, 9d, 91 and 9r of the pulley 11 in a slackened state, only the upward bending wire 8u is pulled, whereby the gap 9c of the rotating body for upward bending (hereinafter, referred to as an upward rotating body) 9u is reduced against an elastic force and a diameter of the rotating body is reduced, and thus, the upward rotating body 9u and the pulley 11 are brought into close contact with each other. Then, resistance occurs between the upward rotating body 9u and the pulley 11, whereby the upward rotating body 9u rotates together with the pulley 11. Consequently, a part of the upward bending wire 8u arranged on the insertion portion 2 side relative to the upward rotating body 9u is pulled and thus moved by rotation of the upward rotating body 9u to start an operation to bend the bending portion 2b upward.

Here, the operator continues to perform an operation to tilt the shaft portion 5a in the same direction via the thumb put on the third operation surface 33 so that the upward rotating body 9u is in close contact with the pulley 11. Then, the upward rotating body 9u in a close-contact state is brought into closer contact with the pulley 11, which is rotated together with the rotating body 9u, and consequently, the part of the upward bending wire 8u arranged on the insertion portion 2 side relative to the upward rotating body 9u is further pulled and moved, whereby the bending portion 2b is further bent upward.

Meanwhile, when the operator continues to press the third operation surface 33 via the cushion of the thumb so as to hold the tilting position of the operation element 5, the force of the close contact between the upward rotating body 9u and the pulley 11 is gradually reduced along with movement of the upward bending wire 8u arranged on the insertion portion 2 side relative to the upward rotating body 9u. Then, the movement of the upward bending wire 8u arranged on the distal end side relative to the upward rotating body 9u is stopped with a tensile force exerted on the upward bending wire 8u.

At this time, the respective bending wires 8d, 81 and 8r are in a slackened state. Accordingly, as a result of the operation element 5 being continuously held in this tilting operation state, the pulled state of the upward bending wire 8u and the slackened state of the bending wires 8d, 81 and 8r are respectively held, whereby the bending portion 2b is held in a bending state corresponding to the tilting operation.

Then, when the operator performs an operation to tilt the operation element 5 to further bend the bending portion 2b in the same direction, the shaft portion 5a is pressed down via the cushion of the thumb put on the third operation surface 33 to perform an operation to further tilt the shaft portion 5a in the same direction. Then, when an operation to further tilt the shaft portion 5a in the same direction, the tilting operation is performed with the cushion and the tip of the thumb put on the third side 5f, which is a side of the third operation surface 33. Then, the bending portion 2b can be brought into a maximum upward bending state by tilting the shaft portion 5a at a maximum angle.

In this case, since the third operation surface 33 has the antislip-finished surface, the thumb is prevented from falling off from the third operation surface 33, enabling a steady operation.

Also, where the operator bends the bending portion 2b in another direction, for example, downward, the cushion of the thumb is put on the fourth operation surface 34 to perform an operation to tilt the shaft portion 5a. Meanwhile, where the operator bends the bending portion 2b in another direction, for example, rightward, a side portion of the thumb is put on the first operation surface 31 to perform an operation to tilt the shaft portion 5a, and where the operator bends the bending portion 2b, for example, leftward, the other side portion of the thumb is put on the second operation surface 32 to perform an operation to tilt the shaft portion 5a.

For bending the bending portion 2b largely downward, the operator puts the tip of the thumb on the fourth side 5g, which is a side of the fourth operation surface 34, to perform a tilting operation. Also, for bending the bending portion 2b largely rightward, the operator puts a side portion of the thumb on the first side 5d, which is the base of the first operation surface 31, to perform a tilting operation. Furthermore, for the bending portion 2b largely leftward, the operator puts the other side portion of the thumb on the first side 5e, which is the base of the second operation surface 32, to perform a tilting operation.

As a result of the operator performing the operation with the thumb put on the respective sides 5d, 5e, 5f and 5g, for example, as indicated by dashed lines in Fig. 7, the upward movement distance increases by Lu, the downward movement distance increases by Ld, the leftward movement distance increases by Ll and the rightward movement distance increases by Lr compared to a case where the finger contact portion 5C has a spherical shape.

With this configuration, the operator puts the cushion or a side portion of the thumb on any of the operation surfaces 31, 32, 33 and 34 of the finger contact portion 5b included in the operation element 5 to perform an operation to tilt the shaft portion 5a, whereby the bending wires 8u, 8d, 81 and 8r can be pulled/slackened to perform an bending operation of the bending portion 2b.

Also, the operator puts the cushion of the thumb or the tip of the thumb on the third side 5f included in the bottom face 30 of the finger contact portion 5b included in the operation element 5 to fall the shaft portion 5a of the operation element 5 down to the farthest from a standing position, whereby a maximum upward tilting operation can easily be performed.

Furthermore, the operator puts the cushion of the thumb or the tip of the thumb on the fourth side 5g included in the bottom face 30 of the finger contact portion 5b included in the operation element 5 to fall the shaft portion 5a of the operation element 5 down to the farthest from the standing position, whereby a maximum downward tilting operation can easily be performed.

Also, a side portion of the thumb is put on the first side 5d included in the bottom face 30 of the finger contact portion 5b included in the operation element 5 to fall the shaft portion 5a of the operation element 5 to the farthest from the standing position, whereby a maximum rightward tilting operation can easily be performed.

Also, a side portion of the thumb is put on the second side 5e included in the bottom face 30 of the finger contact portion 5b included in the operation element 5 to fall the shaft portion 5a of the operation element 5 down to the farthest from the standing position, whereby a maximum leftward tilting operation can easily be performed.

Then, in advance, lengths of the first side 5d, the second side 5e, the third side 5f and the fourth side 5g are set and a position where the attachment portion 5c is formed is set, according to a palm and a thumb of an operator's hand. Consequently, the finger contact portion 5b is provided at a position optimum for the operator, enabling efficient upward/downward tilting operation and leftward/rightward tilting operation. Furthermore, angles and areas of inclined surfaces of the operation surfaces 31, 32, 33 and 34 are set according to the palm and the thumb of the operator, the operator can more steadily perform
upward/downward/leftward/rightward tilting operations.

Furthermore, as a result of the surfaces of the finger contact portion 5b including an elastic member, when a thumb is put on the operation surfaces 31, 32, 33 and 34 to perform an tilting operation of the shaft portion 5a, a load imposed on a side portion of the thumb can be reduced. Consequently, fatigue during bending operation is reduced.

Furthermore, as a result of making a modulus of elasticity of the elastic member provided at the third operation surface 33 or the fourth operation surface 34 on which the cushion of a thumb is put be larger than a modulus of elasticity of the elastic member provided at the first operation surface 31 and the second operation surface 32, operability similar to that of the aforementioned case can be provided. Furthermore, with this configuration, the elastic member of the third operation surface 33 and the fourth operation surface 34 on which a force in a shearing direction relative to the surfaces is easily applied is prevented from being subjected to shear deformation. Consequently, a force applied to the third operation surface 33 and the fourth operation surface 34 is efficiently used as an amount of operation force to perform a tilting operation of the operation element 5.

Also, in the above-described embodiment, the finger contact portion 5b includes an elastic member. However, as illustrated in Fig. 10, a finger contact portion 5b1 may include a core member 5s having rigidity and a covering body 5t made of an elastic member that covers the core member 5s.

The core member 5s has, for example, a circular columnar shape, and includes an attachment portion 5c. The covering body 5t is a pentahedron including a core member arranging hole 5u in which the core member 5s is arranged, and includes a bottom face 30, a first operation surface 31, a second operation surface 32, a third operation surface 33 and a fourth operation surface 34.

As described above, as a result of the finger contact portion 5b1 including the core member 5s and the covering body 5t, the finger contact portion 5b1 can more reliably be fixed to the distal end of the shaft portion 5a. Also, when a problem in operability occurs due to wear of the covering body 5t, the covering body 5t can be removed from the core member 5s, and then replaced with a new covering body 5t by attaching the new covering body 5t to the core member 5s.

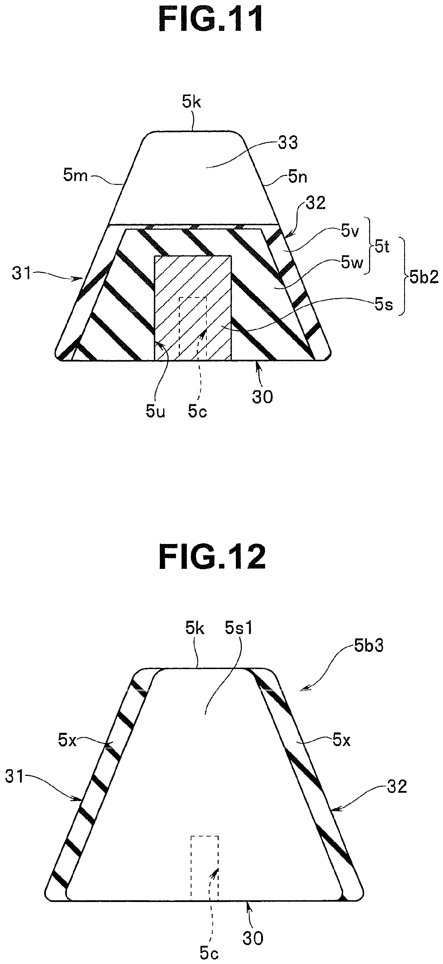

Also, the covering body 5t may be made to have a double-layer structure as illustrated in Fig. 11 to provide a finger contact portion 5b2. In this case, a modulus of elasticity of a first elastic member 5w included in a first layer arranged on the surface side of a core member 5s is set to be larger than a modulus of elasticity of a second elastic member 5v included in a surface layer.

Also, where the covering body 5t includes a plurality of layers, a modulus of elasticity of an elastic member of a first layer is set to be the largest, and the moduli of elasticity of the elastic members are set so that the modulus of elasticity is smaller in order of closeness to the surface layer.

Consequently, the operator can feel a favorable touch, enabling enhancement in operability.

Furthermore, operation and effects similar to the above can also be provided by fixing the first elastic member 5w and the second elastic member 5v via an adhesive having an elastic property.

Furthermore, instead of the finger contact portion 5b and the finger contact portion 5b1 described above, as illustrated in Fig. 12, a finger contact portion 5b3 may be formed using a core member 5s1 and an elastic sheet 5x. The core member 5s1 is a member having rigidity, and has a shape that is substantially the same as the finger contact portion 5b. In other words, the core member 5s1 includes a bottom face 30 including an attachment portion 5c, a first operation surface 31, a second operation surface 32, a third operation surface 33, and a fourth operation surface 34. The elastic sheet 5x, which includes an elastic member, is provided at each of the first operation surface 31 and the second operation surface 32 of the core member 5s1.

With this configuration, when the cushion of a thumb is put on the third operation surface 33 and the fourth operation surface 34 to perform a tilting operation, a force of the thumb is instantly transmitted to the third operation surface 33 and the fourth operation surface 34, providing a favorable operational feeling. Other operation and effects are similar to those of the above-described embodiment.

Also, operability similar to the above can be provided by making a thickness of the elastic member provided at the third operation surface 33 and the fourth operation surface 34 on which the cushion of a thumb is put be smaller than a thickness of the elastic member provided at the first operation surface 31 and the second operation surface 32.

In the above-described embodiment, each of the finger contact portions 5b, 5b1, 5b2 and 5b3 is a pentahedron. However, the finger contact portion is not limited to a pentahedron, and may be a hexahedron as indicated below.

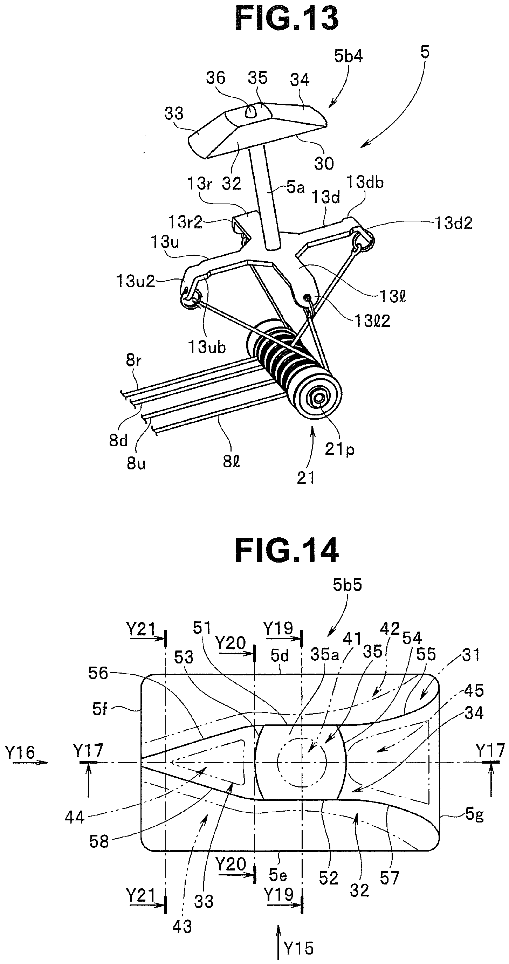

Fig. 13 is a diagram illustrating a finger contact portion according to a second embodiment of the present invention, the finger contact portion being a hexahedron.

As illustrated in Fig. 13, a finger contact portion 5b4 of a operation element 5 according to the present embodiment includes a top face 35 that faces a bottom face 30 and includes, for example, a quadrangular flat surface. In other words, the finger contact portion 5b4 includes the top face 35 instead of the ridge line 5k included in the above-described finger contact portions 5b, 5b1, 5b2 and 5b3.

Consequently, a first operation surface 31 is an inclined surface having a quadrangular shape including a first side 5d of a bottom face 30 and a side that is a first top face portion of the top face 35, the side facing the first side 5d, and likewise, a second operation surface 32 is an inclined surface having a quadrangular shape including a second side 5e of the bottom face 30 and a side that is a second top face portion of the top face 35, the side facing the second side 5e, and likewise, a third operation surface 33 is an inclined surface having a quadrangular shape including a third side 5f of the bottom face 30 and a side that is a third top face portion of the top face 35, the side facing the third side 5f, and likewise, a fourth operation surface 34 is an inclined surface having a quadrangular shape including a fourth side 5g of the bottom face 30 and a side that is a fourth top face portion of the top face 35, the side facing the fourth side 5g. In other words, the first operation surface 31, the second operation surface 32, the third operation surface 33 and the fourth operation surface 34 all have a quadrangular shape.

An elastic member (not illustrated) is provided at least at the first operation surface 31 and the second operation surface 32, and antislip finishing (not illustrated) is provided at least at the third operation surface 33 and the fourth operation surface 34.

The cushion of a thumb is put on the top face 35, the third operation surface 33 and the fourth operation surface 34 of the finger contact portion 5b4, a side portion of the thumb is put on the first operation surface 31, and the other side portion of the thumb is put on the second operation surface 32.

At a center portion of the top face of the finger contact portion 5b4, for example, a projection portion 36 is provided as a finger support portion.

The projection portion 36 is a projection to be supported by the cushion of a thumb. The projection portion 36 has a hemispheric shape having a predetermined diameter dimension or a projection portion set to have a predetermined size and a height dimension, and a distal end shape thereof includes a curve surface or an undulating surface. Here, this projection portion 36 is not limited to one projection portion and may include an aggregate of a plurality of projection portions.

The rest of configuration is similar to those of the above-described finger contact portions 5b, 5b1, 5b2 and 5b3.

As described above, the finger support portion including the projection portion 36 is provided on the top face 35 of the finger contact portion 5b4. Consequently, the projection portion 36 is arranged so that when an operator puts the cushion of his/her thumb on the top face 35, the projection portion 36 digs into the cushion of the thumb, enabling the finger contact portion 5b4 to be held reliably and steadily. Accordingly, when the operator operates the operation element 5 to rotate upward, rightward, downward, leftward, upward, and the like the operator's thumb is prevented from falling off from the top face 35, and thus, can operate the operation element 5 to largely rotate, thereby providing arc motion of a bending portion 2b.

In the above-described embodiment, the top face 35 has a quadrangular shape. However, the shape of the top face 35 is not limited to a quadrangle, and may be a combination of opposed straight lines and curved lines connecting the straight lines or an oval shape or the like including a circular shape. Along with change of the shape of the top face 35 from a quadrangle to a circle or an oval, the first operation surface 31, the second operation surface 32, the third operation surface 33 and the fourth operation surface 34 are also changed in shape to surfaces different from the quadrangular inclined surfaces.

Also, the above-described embodiment has been described in terms of a configuration in which the projection portion 36 is provided at the top face 35 of the finger contact portion 5b4. However, the finger support portion is not limited to a projection portion, and may be a recess portion to be supported as a result of a part of the cushion being received by the recess portion as illustrated in the below-indicated figures. As illustrated in the below-indicated figures, a recess portion may be formed at the operation surfaces 31, 32, 33 and 34 of the finger contact portion 5b4 taking the operability into account. Also, like the projection portion 36, an undulating surface may be formed on an outer surface of this recess portion.

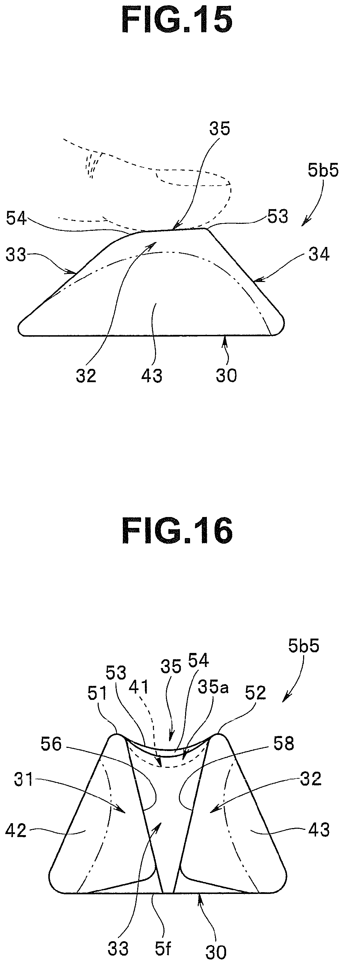

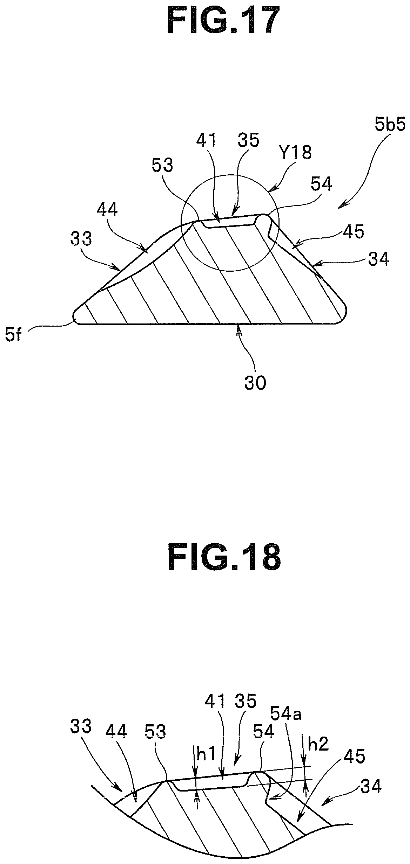

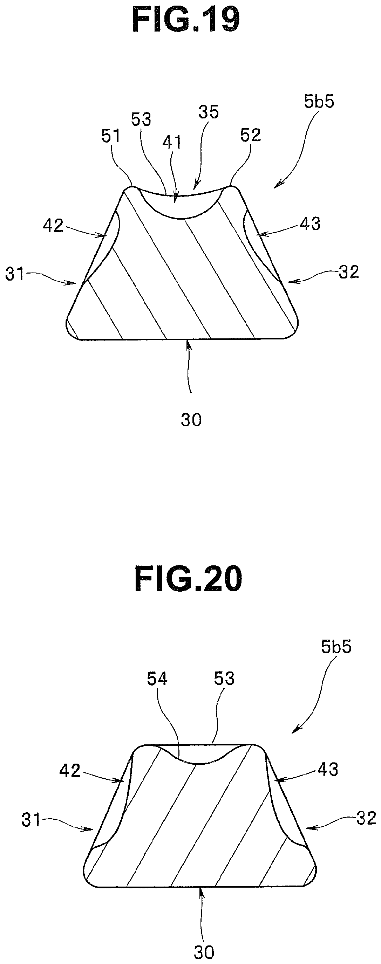

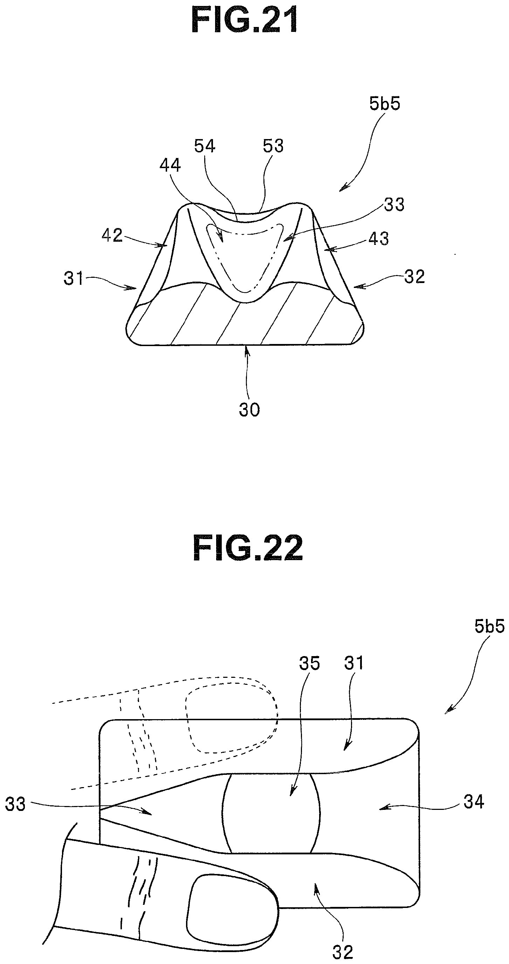

Figs. 14-22 relate to a modification of the finger contact portion configured in the form of a hexahedron, and Fig. 14 is a top view of a finger contact portion including a recess portion on a top face as a finger support portion and including a recess portion each of operation surfaces, Fig. 15 is a side view of the finger contact portion as viewed from the arrow Y15 direction in Fig. 14, Fig. 16 is a back view of the finger contact portion as viewed from the arrow Y16 direction in Fig. 14, Fig. 17 is a cross-sectional diagram along the arrows Y17-Y17 line in Fig. 14, Fig. 18 is an enlarged view of the part indicated by the arrow Y18 in Fig. 17, Fig. 19 is a cross-sectional diagram along the arrows Y19-Y19 line in Fig. 14, Fig. 20 is a cross-sectional diagram along the arrows Y20-Y20 line in Fig. 14, Fig. 21 is a cross-sectional diagram along the arrows Y21-Y21 line in Fig. 14, and Fig. 22 is a diagram illustrating an operational state in which a side portion of a thumb is put on a first operation surface and an operational state in which the other side portion of the thumb is put on a second operation surface.

As illustrated in Figs. 14-16, a finger contact portion 5b5 according to the present embodiment includes a first recess portion 41 at a top face 35, a second recess portion 42 at a first operation surface 31, a third recess portion 43 at a second operation surface 32, a fourth recess portion 44 at a third operation surface 33, and a fourth recess portion 45 at a fourth operation surface 34.

Ridge lines of the respective recess portions 41-45 are each formed by a curve. In Figs. 14-16, ridge lines are formed by the recess portions 41-45 are indicated by alternate long and two short dashed lines. The first recess portion 41 is a recess portion as a finger support.

As indicated by the dashed lines in Fig. 15, the top face 35 is a surface on which the cushion of a thumb is put. As illustrated in Fig. 14, the top face 35 is surrounded by a first ridge line 51, a second ridge line 52 facing the first ridge line 51, and a third ridge line 53 and a fourth ridge line 54 each connecting an end portion of the first ridge line 51 and an end portion of the second ridge line 52, and for example, includes a recessed curved surface 35a (see Fig. 16). At a center portion thereof, as illustrated in Fig. 14, the dashed line in Fig. 16 and Figs. 17-19, the first recess portion 41, which is a finger support portion, is provided. The first recess portion 41 is configured so as to receive a part of the cushion of a thumb.

As illustrated in Fig. 18, a relationship of h1 < h2 is set between a height h1 of a wall from a bottom face of the first recess portion 41 to an upper face of the third ridge line 53 and a height h2 of a wall from the bottom face of the first recess portion 41 to an upper face of the fourth ridge line 54. Also, as illustrated in Fig. 16, a height of a wall from the bottom face of the first recess portion 41 indicated by the dashed line to an upper face of the first ridge line 51 and a height of a wall from the bottom face of the first recess portion 41 to an upper face of the second ridge line 52 are set to be higher than the heights of the walls formed respectively by the third ridge line 53 and the fourth ridge line 54.

In this configuration, with the cushion of a thumb received in the recessed curved surface 35a of the top face 35, an operation to bend a bending portion 2b or an operation to bring the distal end portion 2a into arc motion is performed. During this operation, the operator holds his/her finger against a wall corresponding to a bending direction. Consequently, the operator's finger is reliably prevented from falling off from the first recess portion 41, and a force from the operator is efficiently transmitted to the finger contact portion 5b5. Accordingly, the operator can perform a tilting operation of the shaft portion 5a with small strength.

As described above, in the present embodiment, the finger support portion including the first recess portion 41 is provided at the top face 35 of the finger contact portion 5b4. Consequently, an operator can reliably and steadily hold the finger contact portion 5b4 via his/her thumb to perform a tilting operation of the shaft portion 5a. Accordingly, operation and effects similar to those of the above-described embodiment can be provided.

The recess portions 42-45 of the respective operation surfaces 31-34 will be described with reference to Figs. 14-21.

As illustrated in Fig. 14, the first operation surface 31 is a surface surrounded by a fifth ridge line 55, the first ridge line 51, a sixth ridge line 56, a third side 5f and a first side 5d. The second recess portion 42 illustrated in Figs. 16 and 19-21 is a dent for a side portion of a thumb of an operator to be steadily put thereon. The second recess portion 42 is formed in a predetermined shape at a predetermined position so as to extend from the first operation surface 31 to a side portion of the third operation surface 33.

An operator closely puts a side portion of his/her thumb on a wall of the dent formed by providing the second recess portion 42 at the first operation surface 31 as indicated by the dashed lines in Fig. 22, enabling a rightward bending operation of the shaft portion 5a to be performed steadily. In addition, the operator performs a bending operation with the side portion of the thumb put on the dent, enabling a tilting angle of the shaft portion 5a to be increased compared to that in a bending operation with the cushion of the thumb put on the top face 35.

The fifth ridge line 55 is a ridge line from an intersection between the first side 5d and the fourth side 5g to an intersection between the first ridge line 51 and the fourth ridge line 54. The sixth ridge line 56 is a ridge line from an intersection between the first ridge line 51 and the third ridge line 53 to a predetermined position in the third side 5f.

Meanwhile, as illustrated in Figs. 14 and 15, the second operation surface 32 is a surface surrounded by a seventh ridge line 57, the second ridge line 52, an eighth ridge line 58, the third side 5f and the second side 5e. As illustrated in Figs. 15, 16 and 19-21, the third recess portion 43 is a dent for the other side portion of the thumb of the operator to be steadily put thereon. The third recess portion 43 is formed in a predetermined shape at a predetermined position so as to extend from the second operation surface 32 to a side portion of the third operation surface 33.

Consequently, the operator closely puts the other side portion of the thumb on a wall of the dent formed by providing the third recess portion 43 at the second operation surface 32 as indicated by solid lines in Fig. 22, enabling a leftward bending operation of the shaft portion 5a to be steadily performed. In addition, as a result of the operator performing the bending operation with the other side portion of the thumb put on the dent, the tilting angle of the shaft portion 5a can be increased compared to that in a bending operation with the cushion of the thumb put on the top face 35.

Here, the seventh ridge line 57 is a ridge line from an intersection between the second side 5e and the fourth side 5g to an intersection between the second ridge line 52 and the fourth ridge line 54. The eighth ridge line 58 is a ridge line from an intersection between the second ridge line 52 and the third ridge line 53 to a predetermined position in the third side 5f.

The sixth ridge line 56 and the eighth ridge line 58 described above form an inverted V-shape from the third side 5f toward the top face 35 taking close-contact of a finger into account.

As illustrated in Figs. 14 and 16, the third operation surface 33 is a surface surrounded by the third side 5f, the eighth ridge line 58, the third ridge line 53 and the sixth ridge line 56. As illustrated in Figs. 17 and 21, the third recess portion 43 is formed in a predetermined shape at a predetermined position in the third operation surface 33.

The operator puts the cushion of his/her thumb on the bottom face of the third recess portion 43 formed at the third operation surface 33, enabling an upward bending operation of the shaft portion 5a to steadily be performed. In addition, the operator moves the cushion of the thumb to the third side 5f side along the bottom face of the third recess portion 43 to perform an operation to gradually change the tilting angle of the shaft portion 5a to a maximum tilting angle.

As illustrated in Fig. 14, the fourth operation surface 34 is a surface surrounded by the fourth side 5g, the fifth ridge line 55, the fourth ridge line 54 and the seventh ridge line 57. As illustrated in Fig. 17, the fourth recess portion 45 is formed in a predetermined shape at a predetermined position in the fourth operation surface 34. As a result of forming the fourth recess portion 45 in the fourth operation surface 34, as illustrated in Fig. 18, a rising face 54a from a bottom face of the fourth recess portion 45 to the fourth ridge line 54 is formed.

The operator performs an operation to tilt the finger contact portion 5b5 with the tip of the thumb hooked on the rising face 54a, enabling a downward bending operation of the shaft portion 5a to steadily be performed. In addition, where the operator hooks the cushion of the thumb on the rising face 54a instead of the tip of the thumb hooked on the rising face 54a, the tilting angle of the shaft portion 5a can easily be increased.

A finger contact portion 5b5 optimum for an operator can be provided by setting an angle formed by each of the ridge lines 55 and 57 and the fourth side 5g or an angle formed by each of the ridge lines 56 and 58 and the third side 5f, and shapes and positions of the recess portions 41, 42, 43 and 44 according to a palm and a thumb of the operator's hand.

Also, recess portions 42, 43, 44 and 45 may be provided at the operation surfaces 31, 32, 33 and 34 of the finger contact portion 5b4 or the operation surface 31, 32, 33 and 34 of each of the above-described finger contact portions 5b, 5b1, 5b2 and 5b3 configured in the form of a pentahedron. Consequently, a substantial increase in operability can be achieved.

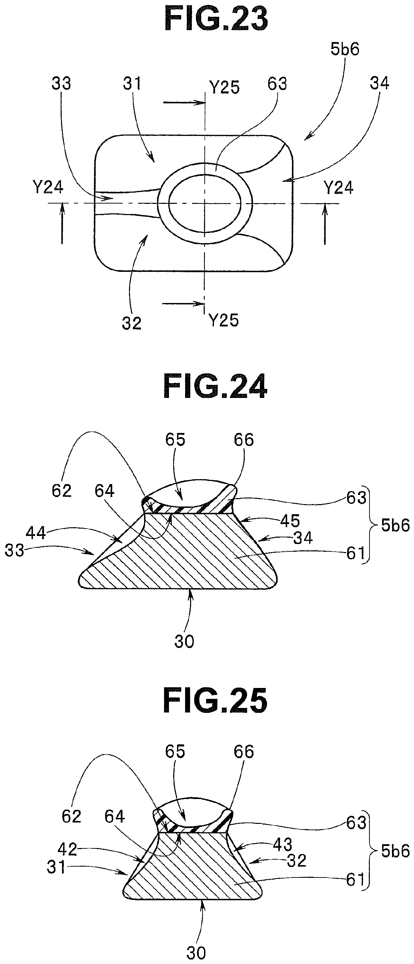

Furthermore, a finger contact portion 5b6 which is illustrated in Figs. 23-27B may be provided.

The finger contact portion 5b6 illustrated in Figs. 23-25 includes a finger rest body 61 and a recess portion-provided finger support member 63.

The finger rest body 61 has, for example, rigidity, and includes a bottom face 30, and a first operation surface 31, a second operation surface 32, a third operation surface 33 and a fourth operation surface 34, which are side portions, and a fixing surface 62. Recess portions 41, 42, 43 and 44 are provided at the first operation surface 31, the second operation surface 32, the third operation surface 33 and the fourth operation surface 34.

The fixing surface 62 is a surface for, e.g., fixing by bonding, on which the recess portion-provided finger support member 63 is arranged and fixed.

Meanwhile, the recess portion-provided finger support member 63 is an elastic member and has a cylindrical shape. The recess portion-provided finger support member 63 includes an attachment surface 64 to be arranged on the fixing surface, and a ring-shaped portion 66 including a finger support recess portion 65. For example, a shape and a height dimension of a rising face of the ring-shaped portion 66 or a diameter dimension of the finger support recess portion 65 are arbitrarily set according to the palm or the thumb of an operator's hand or taking the operability into account.

Also, an undulating antislip member is provided at the finger support recess portion 65, enabling a finger to be prevented from slipping out from the finger support recess portion 65 when the operation element 5 is operated with the finger.

As described above, the finger contact portion 5b6 is formed so as to include the finger rest body 61 and the recess portion-provided finger support member 63, which are separated from each other. Consequently, a finger contact portion 5b6 with a recess portion-provided finger support member 63 formed according to an operator's request attached on a finger rest body 61 can be provided.

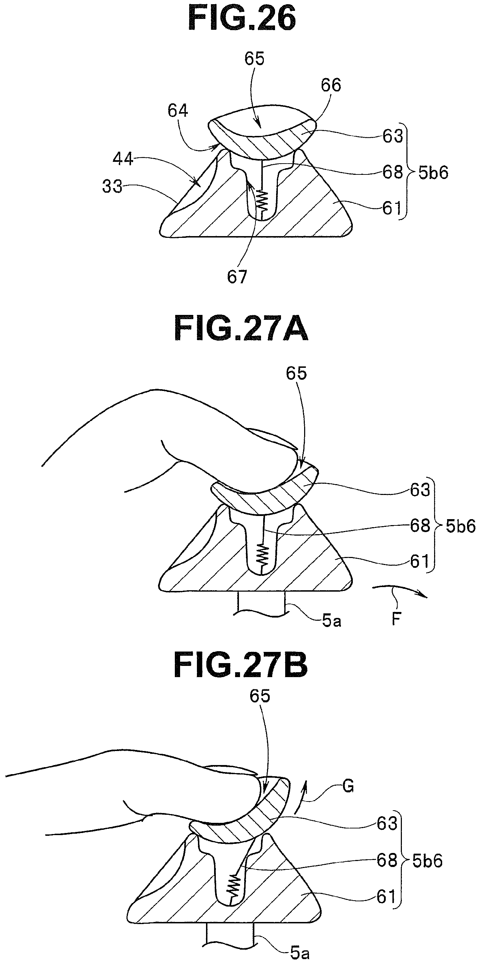

Furthermore, as illustrated in Fig. 26, a hole portion 67 is provided at a predetermined position in a finger rest body 61, an end portion of an end of a spring 68, which is an activation member, is fixed to a bottom face of the hole portion 67. Then, an attachment surface 64 of a recess portion-provided finger support member 63 is fixed at a predetermined position of an end portion of the other end of the spring 68.

Consequently, the recess portion-provided finger support member 63 is slidably attached to the finger rest body 61 via the spring 68 in an integrated manner without falling off from the finger rest body 61.

In the present figure, the attachment surface 64 of the recess portion-provided finger support member 63 is a predetermined curved surface.

With this configuration, as illustrated in Fig. 27A, when a operation element 5 is operated to move in the arrow F direction in the figure with a finger put against a finger support recess portion 65, the attachment surface 64 of the recess portion-provided finger support member 63 slides relative to the finger rest body 61. Consequently, the finger contact portion 5b6 moves as indicated by the arrow G in Fig. 27B. In other words, when the finger contact portion 5b6 is operated, the recess portion-provided finger support member 63 slides, enabling the finger to be prevented from slipping out from the finger support recess portion 65.

Furthermore, provision of an undulating antislip portion and provision of an antislip member at the finger support recess portion 65 enable a finger to be prevented from slipping out from the finger support recess portion 65 when an operation to incline the operation element 5 is performed.

In the above-described embodiments, the bending portion-equipped medical apparatus is an endoscope. However, the bending portion-equipped medical apparatus is not limited to an endoscope, and may be, e.g., a sliding tube used when an endoscope is introduced to an inside of a body or a treatment instrument inserted into a treatment instrument channel of an endoscope.

The present invention is not limited only to the above-described embodiments, and various modifications are possible.

## Claims

1. A bending portion-equipped medical apparatus comprising:
an insertion portion (2) having on a distal end side thereof a bending portion;
an operation portion (3) provided at a proximal end of the insertion portion;
the operation portion includes a grasping portion (3a) and an operation portion body (3b), wherein the grasping portion is consecutively connected to the insertion portion and the operation portion body, the grasping portion having a longitudinal axis which is in a positional relationship that is common or parallel with an insertion axis of the insertion portion, the grasping portion being configured to be grasped by the operator; and
an operation element (5) configured to perform an operation to bend the bending portion, the operation element including a shaft portion (5a) including a distal end portion
and a proximal end portion, the shaft portion being provided to be orthogonal to a longitudinal axis of the operation portion, a tilting direction and an tilting angle of the shaft portion being changeable, and a finger contact portion (5b) fixed to a distal end portion of the shaft portion in an integrated manner;
**characterized in that** the finger contact portion has a pentahedron or hexahedron shape and
wherein the finger contact portion includes:
an attachment surface (30) which is a bottom face having a substantially
quadrangular shape including four sides each set to have a predetermined length, the attachment surface including an attachment portion (5c) to be attached to one end
portion of the shaft portion,
a first operation surface (31) including an inclined surface having a substantially
triangular shape with a first side of the attachment surface as a bottom face of the triangular shape,
a second operation surface (32) including an inclined surface having a substantially
triangular shape with a second side facing the first side as a bottom face of the triangular shape,
a third operation surface (33) including an inclined surface having a substantially
quadrangular shape with a third side intersecting the first side of the attachment surface as a bottom face of the quadrangular shape, and
a fourth operation surface (34) including an inclined surface having a substantially
quadrangular shape with a fourth side facing the third side as a bottom face of the quadrangular shape; and
wherein the third operation surface and the fourth operation surface are arranged on a distal end side and a proximal end side of the longitudinal axis.

2. The bending portion-equipped medical apparatus according to claim 1, comprising a ridge line connecting an apex facing the first side of the first operation surface and an apex facing the second side of the second operation surface.

3. The bending portion-equipped medical apparatus according to claim 2,
wherein a distance between the apex facing the first side of the first operation surface and the apex facing the second side of the second operation surface is set to be shorter than each of a length of the third side and a length of the fourth side or set to be shorter than the length of one of the third side and the fourth side and equal to the length of the other.

4. The bending portion-equipped medical apparatus according to claim 1,
wherein the finger contact portion includes an elastic member having a predetermined resilient property at each operation surface.

5. The bending portion-equipped medical apparatus according to claim 4,
wherein, in a configuration in which an operation to tilt the shaft portion is performed by putting a cushion or a side portion of a thumb of a hand on any of the operation surfaces of the finger contact portion,
the first operation surface and the second operation surface each include a surface on which a side portion of the thumb is put, and
the third operation surface and the fourth operation surface each include a surface on which the cushion of the thumb is put; and
wherein the elastic member is provided at least at the first operation surface and the second operation surface.

6. The bending portion-equipped medical apparatus according to claim 5,
wherein antislip finishing is provided at the third operation surface and the fourth operation surface.

7. The bending portion-equipped medical apparatus according to claim 1,
wherein the finger contact portion includes a core member having rigidity and an elastic member provided on an outer surface side of the core member.

8. The bending portion-equipped medical apparatus according to claim 7,
wherein a modulus of elasticity of the elastic member provided at the third operation surface or the fourth operation surface is larger than a modulus of elasticity of the elastic member provided at the first operation surface and the second operation surface.

9. The bending portion-equipped medical apparatus according to claim 7,
wherein the elastic member has a multi-layer structure at least including a surface layer including in an outermost sheath of the finger contact portion and a first layer arranged on the outer surface of the core member; and
wherein a modulus of elasticity of the elastic member included in the first layer is set to be larger than a modulus of elasticity of the elastic member included in another layer.

10. The bending portion-equipped medical apparatus according to claim 9,
wherein the surface layer and the first layer are fixed to each other via an elastic adhesive.

11. The bending portion-equipped medical apparatus according to claim 1,
wherein, where the finger contact portion has a configuration including a top face having a quadrangular shape or an oval shape, the top face being provided so as to face the attachment surface,
the first operation surface includes a surface including the first side of the attachment surface and a first top face portion that is a part of the top face,
the second operation surface includes a surface including the second side facing the first side and a second top face portion of the top face, the second top face portion being different from the first top face portion,
the third operation surface includes a surface including the third side intersecting the first side of the attachment surface and a third top face portion of the top face, the third top face portion being different from each of the first top face portion and the second top face portion,
the fourth operation surface includes a surface including the fourth side facing the third side and a fourth top face portion of the top face, the fourth top face portion being different from each of the first top face portion, the second top face portion and the third top face portion, and
the finger contact portion includes a finger support portion at the top face.

12. The bending portion-equipped medical apparatus according to claim 11,
wherein the finger support portion includes a projection portion to be supported by a cushion of the thumb, the projection portion being provided at the top face.

13. The bending portion-equipped medical apparatus according to claim 11,
wherein the finger support portion includes a first recess portion that receives a part of a cushion of the thumb, the first recess portion being provided at the top face.

14. The bending portion-equipped medical apparatus according to claim 11,
wherein the finger contact portion includes a finger rest body including a side portion which an operating finger is to be brought into contact with, a recess portion-provided finger support member including a recessed outer surface and an activation member including a fixing portion at respective opposite ends; and
wherein the fixing portion at one end of the activation member is fixed in a hole provided in the finger rest body, and the fixing portion at the other end of the activation member is fixed to an attachment surface of the recess portion-provided finger support member.

15. The bending portion-equipped medical apparatus according to claim 12,
wherein an antislip portion formed by forming an outer surface of the projection portion in an undulated manner, the projection portion being included in the finger support portion, is provided.

16. The bending portion-equipped medical apparatus according to claim 13,
wherein an antislip portion formed by forming an outer surface of the first recess portion in an undulated manner, the first recess portion being included in the finger support portion, is provided.

17. The bending portion-equipped medical apparatus according to claim 11,
wherein, in a configuration in which an operation to tilt the shaft portion is performed by putting a cushion or a side portion of a thumb of a hand on the top face and any of the operation surfaces of the finger contact portion,
each of the first operation surface and the second operation surface is a surface on which a side portion of the thumb is put,
each of the top face, the third operation surface and the fourth operation is a surface on which the cushion of the thumb is put, and
an elastic member is provided at least at the first finger operation surface and the second operation surface.

18. The bending portion-equipped medical apparatus according to claim 17,
wherein the finger contact portion includes a second recess portion on which a side portion of the thumb is put, the second recess portion being provided from the first operation surface to the third operation surface, and a third recess portion on which the other side portion of the thumb is put, the third recess portion being provided from the second operation surface to the third operation surface.

19. The bending portion-equipped medical apparatus according to claim 18,
wherein the finger contact portion includes a fourth recess portion on which a distal end side of the cushion of the thumb is put, at the fourth operation surface.

20. The bending portion-equipped medical apparatus according to claim 18,
wherein the finger contact portion includes a fifth recess portion on which the cushion of the thumb is put, at the third operation surface.

21. The bending portion-equipped medical apparatus according to claim 18,
wherein the second recess portion and the third recess portion are each arranged so as to have an inverted V-shape from the third side toward the top face.

22. The bending portion-equipped medical apparatus according to claim 1,
wherein the other end portion of the shaft portion is fixed on a center axis of a hanging frame to which an end portion of each pair of pulling members extending from the bending portion is fixed.

## Patentansprüche

1. Mit einem Biegeabschnitt ausgestattetes medizinisches Gerät, das umfasst:
- einen Einführabschnitt (2), der auf einer distalen Endseite einen Biegeabschnitt aufweist,
- einen Bedienabschnitt (3), der an einem proximalen Ende des Einführabschnitts bereitgestellt ist,
- wobei der Bedienabschnitt einen Greifabschnitt (3a) und einen Bedienabschnittskörper (3b) umfasst, wobei der Greifabschnitt nacheinander mit dem Einführabschnitt und dem Bedienabschnittskörper verbunden ist, der Greifabschnitt eine Längsachse hat, die sich in einer gemeinsamen oder parallelen Lagebeziehung zu einer Einführachse des Einführabschnitts befindet, und der Greifabschnitt dazu eingerichtet ist, von einem Bediener ergriffen zu werden, und
- ein Bedienelement (5), das dazu eingerichtet ist, einen Betrieb zum Biegen des Biegeabschnitts durchführen, wobei das Bedienelement einen Schaftabschnitt (5a) mit einem distalen Endabschnitt und einem proximalen Endabschnitt umfasst, wobei der Schaftabschnitt orthogonal zu einer Längsachse des Bedienabschnitts bereitgestellt ist, eine Kipprichtung und ein Kippwinkel des Schaftabschnitts veränderbar sind und ein Fingerkontaktabschnitt (5b) integriert an einem distalen Endabschnitt des Schaftabschnitts befestigt ist,
- **dadurch gekennzeichnet, dass** der Fingerkontaktabschnitt eine fünfflächige oder sechsflächige Form hat,
- wobei der Fingerkontaktabschnitt umfasst:
- eine Befestigungsfläche (30), die eine Unterseite mit einer im Wesentlichen viereckigen Form ist, die vier jeweils auf eine vorgegebene Länge festgelegte Seiten umfasst, wobei die Befestigungsfläche einen Befestigungsabschnitt (5c) aufweist, der an einem Endabschnitt des Schaftabschnitts zu befestigen ist,
- eine erste Bedienfläche (31), die eine geneigte Oberfläche mit einer im Wesentlichen dreieckigen Form umfasst, wobei eine erste Seite der Befestigungsfläche eine Unterseite der dreieckigen Form darstellt,
- eine zweite Bedienfläche (32), die eine geneigte Oberfläche mit einer im Wesentlichen dreieckigen Form umfasst, wobei eine der ersten Seite zugewandte zweite Seite eine Unterseite der dreieckigen Form darstellt,
- eine dritte Bedienfläche (33), die eine geneigte Oberfläche mit einer im Wesentlichen viereckigen Form umfasst, wobei eine dritte Seite, die die erste Seite der Befestigungsfläche schneidet, eine Unterseite der viereckigen Form darstellt, und
- eine vierte Bedienfläche (34), die eine geneigte Oberfläche mit einer im Wesentlichen viereckigen Form umfasst, wobei eine der dritten Seite zugewandte vierte Seite eine Unterseite der viereckigen Form darstellt, und
- wobei die dritte Bedienfläche und die vierte Bedienfläche auf einer distalen Endseite und einer proximalen Endseite der Längsachse angeordnet sind.

2. Mit einem Biegeabschnitt ausgestattetes medizinisches Gerät nach Anspruch 1, das eine Gratlinie umfasst, die einen der ersten Seite der ersten Bedienfläche zugewandten Scheitelpunkt und einen der zweiten Seite der zweiten Bedienfläche zugewandten Scheitelpunkt miteinander verbindet.

3. Mit einem Biegeabschnitt ausgestattetes medizinisches Gerät nach Anspruch 2, wobei ein Abstand zwischen dem der ersten Seite der ersten Bedienfläche zugewandten Scheitelpunkt und dem der zweiten Seite der zweiten Bedienfläche zugewandten Scheitelpunkt kürzer als jeweils eine Länge der dritten Seite und eine Länge der vierten Seite oder kürzer als die Länge der dritten oder vierten Seite und gleich der Länge der anderen festgelegt ist.

4. Mit einem Biegeabschnitt ausgestattetes medizinisches Gerät nach Anspruch 1, wobei der Fingerkontaktabschnitt an jeder Bedienfläche ein elastisches Element mit einer vorgegebenen nachgiebigen Eigenschaft umfasst.

5. Mit einem Biegeabschnitt ausgestattetes medizinisches Gerät nach Anspruch 4, wobei,
- bei einer Konfiguration, bei der ein Betrieb zum Kippen des Schaftabschnitts durch Auflegen eines Kissens oder Seitenabschnitts eines Daumens einer Hand auf eine beliebige der Bedienflächen des Fingerkontaktabschnitts durchgeführt wird,
- die erste Bedienfläche und die zweite Bedienfläche jeweils eine Oberfläche umfassen, auf die ein Seitenabschnitt des Daumens aufgelegt wird, und
- die dritte Bedienfläche und die vierte Bedienfläche jeweils eine Oberfläche umfassen, auf die das Kissen des Daumens aufgelegt wird, und
- das elastische Element zumindest an der ersten Bedienfläche und der zweiten Bedienfläche bereitgestellt ist.

6. Mit einem Biegeabschnitt ausgestattetes medizinisches Gerät nach Anspruch 5, wobei an der dritten Bedienfläche und der vierten Bedienfläche eine rutschfeste Oberflächenbeschaffenheit vorgesehen ist.

7. Mit einem Biegeabschnitt ausgestattetes medizinisches Gerät nach Anspruch 1, wobei der Fingerkontaktabschnitt ein steifes Kernelement und ein an einer Außenflächenseite des Kernelements bereitgestelltes elastisches Element umfasst.

8. Mit einem Biegeabschnitt ausgestattetes medizinisches Gerät nach Anspruch 7, wobei ein Elastizitätsmodul des an der dritten Bedienfläche oder vierten Bedienfläche bereitgestellten elastischen Elements größer ist als ein Elastizitätsmodul des an der ersten Bedienfläche und zweiten Bedienfläche bereitgestellten elastischen Elements.

9. Mit einem Biegeabschnitt ausgestattetes medizinisches Gerät nach Anspruch 7,
- wobei das elastische Element einen mehrschichtigen Aufbau hat, der wenigstens eine Oberflächenschicht, die eine äußerste Umhüllung des Fingerkontaktabschnitts umfasst, und eine erste Schicht enthält, die an der Außenfläche des Kernelements angeordnet ist, und
- wobei ein Elastizitätsmodul des in der ersten Schicht enthaltenen elastischen Elements größer als ein Elastizitätsmodul des in einer anderen Schicht enthaltenen elastischen Elements festgelegt ist.

10. Mit einem Biegeabschnitt ausgestattetes medizinisches Gerät nach Anspruch 9, wobei die Oberflächenschicht und die erste Schicht durch einen elastischen Klebstoff aneinander befestigt sind.

11. Mit einem Biegeabschnitt ausgestattetes medizinisches Gerät nach Anspruch 1, wobei,
- wenn der Fingerkontaktabschnitt eine Konfiguration hat, die eine Oberseite mit einer viereckigen Form oder ovalen Form umfasst, die Oberseite so bereitgestellt ist, dass sie der Befestigungsfläche zugewandt ist,
- die erste Bedienfläche eine Oberfläche umfasst, die die erste Seite der Befestigungsfläche und einen ersten Oberseitenabschnitt umfasst, der Teil der Oberseite ist,
- die zweite Bedienfläche eine Oberfläche umfasst, die die der ersten Seite zugewandte zweite Seite und einen zweiten Oberseitenabschnitt der Oberseite umfasst, wobei sich der zweite Oberseitenabschnitt vom ersten Oberseitenabschnitt unterscheidet,
- die dritte Bedienfläche eine Oberfläche umfasst, die die dritte Seite, welche die erste Seite der Befestigungsfläche schneidet, und einen dritten Oberseitenabschnitt der Oberseite umfasst, wobei sich der dritte Oberseitenabschnitt jeweils vom ersten Oberseitenabschnitt und zweiten Oberseitenabschnitt unterscheidet,
- die vierte Bedienfläche eine Oberfläche umfasst, die die der dritten Seite zugewandte vierte Seite und einen vierten Oberseitenabschnitt der Oberseite umfasst, wobei sich der vierte Oberseitenabschnitt jeweils vom ersten Oberseitenabschnitt, zweiten Oberseitenabschnitt und dritten Oberseitenabschnitt unterscheidet, und
- der Fingerkontaktabschnitt an der Oberseite einen Fingerhalteabschnitt umfasst.

12. Mit einem Biegeabschnitt ausgestattetes medizinisches Gerät nach Anspruch 11, wobei der Fingerhalteabschnitt einen von einem Kissen des Daumens zu haltenden vorspringenden Abschnitt umfasst, wobei der vorspringende Abschnitt an der Oberseite bereitgestellt ist.

13. Mit einem Biegeabschnitt ausgestattetes medizinisches Gerät nach Anspruch 11, wobei der Fingerhalteabschnitt einen ersten Aussparungsabschnitt umfasst, der einen Teil eines Kissens des Daumens aufnimmt, wobei der erste Aussparungsabschnitt an der Oberseite bereitgestellt ist.

14. Mit einem Biegeabschnitt ausgestattetes medizinisches Gerät nach Anspruch 11,
- wobei der Fingerkontaktabschnitt einen Fingerauflagekörper mit einem Seitenabschnitt, mit dem ein Bedienfinger in Kontakt zu bringen ist, ein mit einem Aussparungsabschnitt versehenes Fingerhalteelement mit einer ausgesparten Außenfläche und ein Aktivierungselement umfasst, das an jeweiligen entgegengesetzten Enden einen Befestigungsabschnitt aufweist, und
- wobei der Befestigungsabschnitt an einem Ende des Aktivierungselements in einem im Fingerauflagekörper vorgesehenen Loch befestigt ist und der Befestigungsabschnitt am anderen Ende des Aktivierungselements an einer Befestigungsfläche des mit dem Aussparungsabschnitt versehenen Fingerhalteelements befestigt ist.

15. Mit einem Biegeabschnitt ausgestattetes medizinisches Gerät nach Anspruch 12, wobei ein rutschfester Abschnitt bereitgestellt ist, der durch welliges Ausbilden einer Außenfläche des vorspringenden Abschnitts gebildet wird, wobei der vorspringende Abschnitt im Fingerhalteabschnitt enthalten ist.

16. Mit einem Biegeabschnitt ausgestattetes medizinisches Gerät nach Anspruch 13, wobei ein rutschfester Abschnitt bereitgestellt ist, der durch welliges Ausbilden einer Außenfläche des ersten Aussparungsabschnitt gebildet wird, wobei der erste Aussparungsabschnitt im Fingerhalteabschnitt enthalten ist.

17. Mit einem Biegeabschnitt ausgestattetes medizinisches Gerät nach Anspruch 11, wobei,
- bei einer Konfiguration, bei der ein Betrieb zum Kippen des Schaftabschnitts durch Auflegen eines Kissens oder Seitenabschnitts eines Daumens einer Hand auf die Oberseite und eine beliebige der Bedienflächen des Fingerkontaktabschnitts durchgeführt wird,
- die erste Bedienfläche und die zweite Bedienfläche jeweils eine Oberfläche sind, auf die ein Seitenabschnitt des Daumens aufgelegt wird,
- die Oberseite, die dritte Bedienfläche und die vierte Bedienfläche jeweils eine Oberfläche sind, auf die das Kissen des Daumens aufgelegt wird, und
- ein elastisches Element zumindest an der ersten Fingerbedienfläche und der zweiten Bedienfläche bereitgestellt ist.

18. Mit einem Biegeabschnitt ausgestattetes medizinisches Gerät nach Anspruch 17, wobei der Fingerkontaktabschnitt einen zweiten Aussparungsabschnitt, auf den ein Seitenabschnitt des Daumens aufgelegt wird, wobei der zweite Aussparungsabschnitt von der ersten Bedienfläche zur dritten Bedienfläche bereitgestellt ist, und einen dritten Aussparungsabschnitt umfasst, auf den der andere Seitenabschnitt des Daumens aufgelegt wird, wobei der dritte Aussparungsabschnitt von der zweiten Bedienfläche zur dritten Bedienfläche bereitgestellt ist.

19. Mit einem Biegeabschnitt ausgestattetes medizinisches Gerät nach Anspruch 18, wobei der Fingerkontaktabschnitt an der vierten Bedienfläche einen vierten Aussparungsabschnitt umfasst, auf den eine distale Endseite des Kissens des Daumens aufgelegt wird.

20. Mit einem Biegeabschnitt ausgestattetes medizinisches Gerät nach Anspruch 18, wobei der Fingerkontaktabschnitt an der dritten Bedienfläche einen fünften Aussparungsabschnitt umfasst, auf den das Kissen des Daumens aufgelegt wird.

21. Mit einem Biegeabschnitt ausgestattetes medizinisches Gerät nach Anspruch 18, wobei der zweite Aussparungsabschnitt und der dritte Aussparungsabschnitt jeweils so angeordnet sind, dass sie von der dritten Seite zur Oberseite eine umgekehrte V-Form haben.

22. Mit einem Biegeabschnitt ausgestattetes medizinisches Gerät nach Anspruch 1, wobei der andere Endabschnitt des Schaftabschnitts an einer Mittelachse eines Hängerahmens befestigt ist, an dem ein Endabschnitt jedes Paares Zugelemente befestigt ist, das sich vom Biegeabschnitt erstreckt.

## Revendications

1. Appareil médical équipé d'une partie de flexion comprenant :
une partie d'insertion (2) ayant sur un côté d'extrémité distale de celle-ci une partie de flexion ;
une partie d'opération (3) prévue à une extrémité proximale de la partie d'insertion ;
la partie d'opération inclut une partie de préhension (3a) et un corps (3b) de partie d'opération, dans lequel la partie de préhension est consécutivement connectée à la partie d'insertion et au corps de partie d'opération, la partie de préhension ayant un axe longitudinal qui est dans une relation de position qui est commune à ou parallèle avec un axe d'insertion de la partie d'insertion, la partie de préhension étant configurée pour être prise en main par l'opérateur ; et
un élément d'opération (5) configuré pour exécuter une opération pour fléchir la partie de flexion, l'élément d'opération incluant une partie d'arbre (5a) incluant une partie d'extrémité distale et une partie d'extrémité proximale, la partie d'arbre étant prévue pour être orthogonale à un axe longitudinal de la partie d'opération, un sens de basculement et un angle de basculement de la partie d'arbre pouvant être changés, et une partie de contact de doigt (5b) fixée à une partie d'extrémité distale de la partie d'arbre d'une manière intégrée ;
**caractérisé en ce que** la partie de contact de doigt a une forme de pentaèdre ou d'hexaèdre et
dans lequel la partie de contact de doigt inclut :
une surface de fixation (30) qui est une face inférieure ayant une forme sensiblement quadrangulaire incluant quatre côtés chacun défini pour avoir une longueur prédéterminée, la surface de fixation incluant une partie de fixation (5c) destinée à être fixée à une partie d'extrémité de la partie d'arbre,
une première surface d'opération (31) incluant une surface inclinée ayant une forme sensiblement triangulaire avec un premier côté de la surface de fixation comme une face inférieure de la forme triangulaire,
une deuxième surface d'opération (32) incluant une surface inclinée ayant une forme sensiblement triangulaire avec un deuxième côté faisant face au premier côté comme une face inférieure de la forme triangulaire,
une troisième surface d'opération (33) incluant une surface inclinée ayant une forme sensiblement quadrangulaire avec un troisième côté coupant le premier côté de la surface de fixation comme une face inférieure de la forme quadrangulaire, et
une quatrième surface d'opération (34) incluant une surface inclinée ayant une forme sensiblement quadrangulaire avec un quatrième côté faisant face au troisième côté comme une face inférieure de la forme quadrangulaire ; et
dans lequel la troisième surface d'opération et la quatrième surface d'opération sont agencées sur un côté d'extrémité distale et un côté d'extrémité proximale de l'axe longitudinal.

2. Appareil médical équipé d'une partie de flexion selon la revendication 1,
comprenant une ligne de crête connectant un sommet faisant face au premier côté de la première surface d'opération et un sommet faisant face au deuxième côté de la deuxième surface d'opération.

3. Appareil médical équipé d'une partie de flexion selon la revendication 2,
dans lequel une distance entre le sommet faisant face au premier côté de la première surface d'opération et le sommet faisant face au deuxième côté de la deuxième surface d'opération est définie pour être plus courte que chacune d'une longueur du troisième côté et d'une longueur du quatrième côté ou définie pour être plus courte que la longueur d'un du troisième côté et du quatrième côté et égale à la longueur de l'autre.

4. Appareil médical équipé d'une partie de flexion selon la revendication 1,
dans lequel la partie de contact de doigt inclut un élément élastique ayant une propriété résiliente prédéterminée au niveau de chaque surface d'opération.

5. Appareil médical équipé d'une partie de flexion selon la revendication 4,
dans lequel, dans une configuration dans laquelle une opération pour basculer la partie d'arbre est mise en oeuvre en mettant un coussinet ou une partie latérale d'un pouce d'une main sur l'une quelconque des surfaces d'opération de la partie de contact de doigt,
la première surface d'opération et la deuxième surface d'opération incluent chacune une surface sur laquelle une partie latérale du pouce est posée, et
la troisième surface d'opération et la quatrième surface d'opération incluent chacune une surface sur laquelle le coussinet du pouce est posé ; et
dans lequel l'élément élastique est prévu au moins au niveau de la première surface d'opération et de la deuxième surface d'opération.

6. Appareil médical équipé d'une partie de flexion selon la revendication 5,
dans lequel une finition antidérapante est prévue au niveau de la troisième surface d'opération et de la quatrième surface d'opération.

7. Appareil médical équipé d'une partie de flexion selon la revendication 1,
dans lequel la partie de contact de doigt inclut un élément de coeur ayant une rigidité et un élément élastique prévu sur un côté de surface extérieure de l'élément de coeur.

8. Appareil médical équipé d'une partie de flexion selon la revendication 7,
dans lequel un module d'élasticité de l'élément élastique prévu au niveau de la troisième surface d'opération ou de la quatrième surface d'opération est supérieur à un module d'élasticité de l'élément élastique prévu au niveau de la première surface d'opération et de la deuxième surface d'opération.

9. Appareil médical équipé d'une partie de flexion selon la revendication 7,
dans lequel l'élément élastique a une structure multicouches incluant au moins une couche de surface incluant une gaine la plus extérieure de la partie de contact de doigt et une première couche agencée sur la surface extérieure de l'élément de coeur ; et
dans lequel un module d'élasticité de l'élément élastique inclus dans la première couche est défini pour être supérieur à un module d'élasticité de l'élément élastique inclus dans une autre couche.

10. Appareil médical équipé d'une partie de flexion selon la revendication 9,
dans lequel la couche de surface et la première couche sont fixées l'une à l'autre par l'intermédiaire d'un adhésif élastique.

11. Appareil médical équipé d'une partie de flexion selon la revendication 1,
dans lequel, si la partie de contact de doigt a une configuration incluant une face supérieure ayant une forme quadrangulaire ou une forme ovale, la face supérieure étant prévue de façon à faire face à la surface de fixation,
la première surface d'opération inclut une surface incluant le premier côté de la surface de fixation et une première partie de face supérieure qui est une partie de la face supérieure,
la deuxième surface d'opération inclut une surface incluant le deuxième côté faisant face au premier côté et une deuxième partie de face supérieure de la face supérieure, la deuxième partie de face supérieure étant différente de la première partie de face supérieure,
la troisième surface d'opération inclut une surface incluant le troisième côté coupant le premier côté de la surface de fixation et une troisième partie de face supérieure de la face supérieure, la troisième partie de face supérieure étant différente de chacune de la première partie de face supérieure et de la deuxième partie de face supérieure,
la quatrième surface d'opération inclut une surface incluant le quatrième côté faisant face au troisième côté et une quatrième partie de face supérieure de la face supérieure, la quatrième partie de face supérieure étant différente de chacune de la première partie de face supérieure, de la deuxième partie de face supérieure et de la troisième partie de face supérieure, et
la partie de contact de doigt inclut une partie de support de doigt au niveau de la face supérieure.

12. Appareil médical équipé d'une partie de flexion selon la revendication 11,
dans lequel la partie de support de doigt inclut une partie de projection destinée à être supportée par un coussinet du pouce, la partie de projection étant prévue au niveau de la face supérieure.

13. Appareil médical équipé d'une partie de flexion selon la revendication 11,
dans lequel la partie de support de doigt inclut une première partie en renfoncement qui reçoit une partie d'un coussinet du pouce, la première partie en renfoncement étant prévue au niveau de la face supérieure.

14. Appareil médical équipé d'une partie de flexion selon la revendication 11,
dans lequel la partie de contact de doigt inclut un corps de repos de doigt incluant une partie latérale avec laquelle un doigt opérationnel doit être mis en contact, un élément de support de doigt prévu avec une partie en renfoncement incluant une surface extérieure en renfoncement et un élément d'activation incluant une partie de fixation à des extrémités opposées respectives ; et
dans lequel la partie de fixation à une extrémité de l'élément d'activation est fixée dans un trou prévu dans le corps de repos de doigt, et la partie de fixation à l'autre extrémité de l'élément d'activation est fixée à une surface de fixation de l'élément de support de doigt prévu avec une partie en renfoncement.

15. Appareil médical équipé d'une partie de flexion selon la revendication 12,
dans lequel une partie antidérapante formée en formant une surface extérieure de la partie de projection d'une manière ondulée, la partie de projection étant incluse dans la partie de support de doigt, est prévue.

16. Appareil médical équipé d'une partie de flexion selon la revendication 13,
dans lequel une partie antidérapante formée en formant une surface extérieure de la première partie en renfoncement d'une manière ondulée, la première partie en renfoncement étant incluse dans la partie de support de doigt, est prévue.

17. Appareil médical équipé d'une partie de flexion selon la revendication 11,
dans lequel, dans une configuration dans laquelle une opération pour basculer la partie d'arbre est mise en oeuvre en posant un coussinet ou une partie latérale d'un pouce d'une main sur la face supérieure et l'une quelconque des surfaces d'opération de la partie de contact de doigt,
chacune de la première surface d'opération et de la deuxième surface d'opération est une surface sur laquelle une partie latérale du pouce est posée,
chacune de la face supérieure, de la troisième surface d'opération et de la quatrième surface d'opération est une surface sur laquelle le coussinet du pouce est posé, et
un élément élastique est prévu au moins au niveau de la première surface d'opération de doigt et de la deuxième surface d'opération.

18. Appareil médical équipé d'une partie de flexion selon la revendication 17,
dans lequel la partie de contact de doigt inclut une deuxième partie en renfoncement sur laquelle une partie latérale du pouce est posée, la deuxième partie en renfoncement étant prévue de la première surface d'opération jusqu'à la troisième surface d'opération, et une troisième partie en renfoncement sur laquelle l'autre partie latérale du pouce est posée, la troisième partie en renfoncement étant prévue de la deuxième surface d'opération jusqu'à la troisième surface d'opération.

19. Appareil médical équipé d'une partie de flexion selon la revendication 18,
dans lequel la partie de contact de doigt inclut une quatrième partie en renfoncement sur laquelle un côté d'extrémité distale du coussinet du pouce est posé, au niveau de la quatrième surface d'opération.

20. Appareil médical équipé d'une partie de flexion selon la revendication 18,
dans lequel la partie de contact de doigt inclut une cinquième partie en renfoncement sur laquelle le coussinet du pouce est posé, au niveau de la troisième surface d'opération.

21. Appareil médical équipé d'une partie de flexion selon la revendication 18,
dans lequel la deuxième partie en renfoncement et la troisième partie en renfoncement sont chacune agencées de manière à avoir une forme en V inversé depuis le troisième côté vers la face supérieure.

22. Appareil médical équipé d'une partie de flexion selon la revendication 1,
dans lequel l'autre partie d'extrémité de la partie d'arbre est fixée sur un axe central d'un châssis en suspension sur lequel une partie d'extrémité de chaque paire d'éléments de traction s'étendant depuis la partie de flexion est fixée.
